(19) Europäisches Patentamt
European Patent Office
Office européen des brevets

(11) Publication number: **0 386 940 A1**

(12) **EUROPEAN PATENT APPLICATION**

(21) Application number: **90302187.1**

(51) Int. Cl.[5]: **C07D 499/68, C07C 251/48, A61K 31/43**

(22) Date of filing: **01.03.90**

The title of the invention has been amended (Guidelines for Examination in the EPO, A-III, 7.3).

(30) Priority: **07.03.89 GB 8905160**

(43) Date of publication of application: **12.09.90 Bulletin 90/37**

(84) Designated Contracting States: **AT BE CH DE DK ES FR GB GR IT LI LU NL SE**

(71) Applicant: **BEECHAM GROUP PLC**
**Beecham House Great West Road**
**Brentford Middlesex TW8 9BD(GB)**

(72) Inventor: **Bicknell, Andrew John, Beecham Pharmaceuticals**
**Research Division, Brockham Park**
**Betchworth, Surrey RH3 7AJ(GB)**
Inventor: **Gasson, Brian Charles, Beecham Pharmaceuticals**
**Research Division, Brockham Park**
**Betchworth, Surrey RH3 7AJ(GB)**
Inventor: **Hardy, Kenneth David, Beecham Pharmaceuticals**
**Research Division, Brockham Park**
**Betchworth, Surrey RH3 7AJ(GB)**

(74) Representative: **West, Vivien et al**
**Beecham Pharmaceuticals Patents & Trade Marks Department Great Burgh Yew Tree Bottom Road**
**GB-Epsom, Surrey KT18 5XQ(GB)**

(54) **6-Beta-(alpha-etherified oxymino)-acyl amino penicillins.**

(57) Compounds of formula (I):

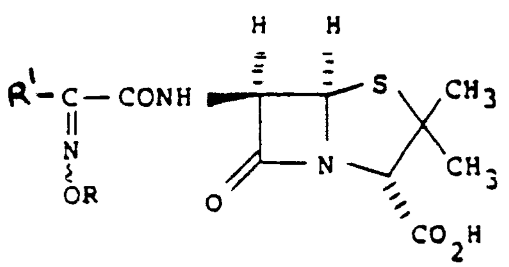

(I)

wherein $R^1$ is optionally substituted phenyl and R is a cycloalkyl group having an alkyl substituent in the 1-position; or $R^1$ is phenyl substituted by at least one fluorine atom and/or at least one trifluoromethyl group and R is hydrogen or an organic radical, and their salts and esters, are useful in the treatment of bacterial infections in humans and animals.

EP 0 386 940 A1

## NOVEL COMPOUNDS

This invention relates to novel β-lactam containing compounds, their preparation and their use, and in particular to a novel class of penicillins. These compounds have antibacterial properties, and therefore are of use in the treatment of bacterial infections in humans and animals caused by a wide range of organisms.

British Patent Specification 1 399 087 discloses a novel class of penicillin antibiotics containing a 6β-(α-etherified oxyimino)-acylamino group.

We have now discovered a particular class of penicillins containing a 6β-(α-etherified oxyimino)-acylamino group that possess good antibacterial activity and a high level of stability to bacterial β-lactamases. Certain compounds of the present invention are also characterized by high blood levels after oral administration.

The present invention provides a compound of formula (I) or a pharmaceutically acceptable salt or in-vivo hydrolysable ester thereof:

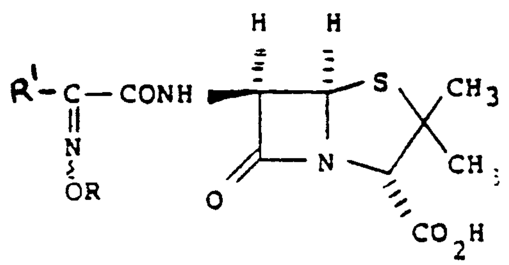

(I)

wherein $R^1$ is optionally substituted phenyl and R is a cycloalkyl group having an alkyl substituent in the 1-position; or $R^1$ is phenyl substituted by at least one fluorine atom and/or at least one trifluoromethyl group and R is hydrogen or an organic radical.

Suitable substituents for phenyl include halogen, $C_{1-6}$ alkyl, phenyl, $C_{1-6}$ alkoxy, halo($C_{1-6}$) alkyl, hydroxy, amino, nitro, carboxy, $C_{1-6}$ alkoxycarbonyl, $C_{1-6}$ alkoxycarbonyl-($C_{1-6}$)-alkyl, $C_{1-6}$ alkylcarbonyloxy, and $C_{1-6}$ alkylcarbonyl.

$R^1$ is preferably phenyl substituted by at least one fluorine atom and/or at least one trifluoromethyl group, and optionally substituted by up to four additional substitutents.

A particular value of $R^1$ within the present invention is 2-fluorophenyl.

Suitable organic radicals include alkyl and cycloalkyl.

As used herein, alkyl includes straight or branched $C_{1-12}$ alkyl, more particularly straight or branched $C_{1-6}$ alkyl, such as methyl or ethyl. Cycloalkyl includes $C_{3-12}$, more particularly $C_{5-8}$, especially $C_{5-7}$ cycloalkyl.

In a preferred aspect, R is a $C_{5-8}$, more preferably $C_{5-7}$, cycloalkyl group having a $C_{1-6}$ alkyl substituent in the 1-position, more particularly methyl or ethyl, preferably methyl.

Advantageously R is 1-methylcyclopentyl or 1-methylcyclohexyl.

An alkyl or cycloalkyl group may contain one or more optional substituents. For example, when group R is a cycloalkyl group having an alkyl substituent in the 1-position, it may contain one or more optional additional substituents on the cycloalkyl moiety thereof and/or one or more optional substituents on the 1-alkyl moiety thereof.

Suitable substituents for an alkyl or cycloalkyl group include halogen, $C_{1-6}$ alkoxy, carboxy and esters, amides and salts thereof, $C_{1-6}$ alkanoyloxy and hydroxy. Suitable substituents for a cycloalkyl group additionally include $C_{1-6}$ alkyl, oxo, hydroxyimino, $C_{1-6}$ alkoxyimino and methylene optionally substituted by, for example, one or two halogen atoms. Halogen includes fluorine, chlorine, bromine and iodine.

Examples of suitable pharmaceutically acceptable in vivo hydrolysable ester groups include those which break down readily in the human body to leave the parent acid or its salt. Suitable ester groups of this type include those of part formula (i), (ii), (iii) and (iv):

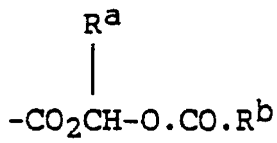

(i)

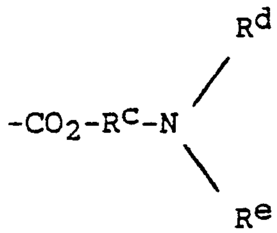

(ii)

$-CO_2CH_2-OR^f$ (iii)

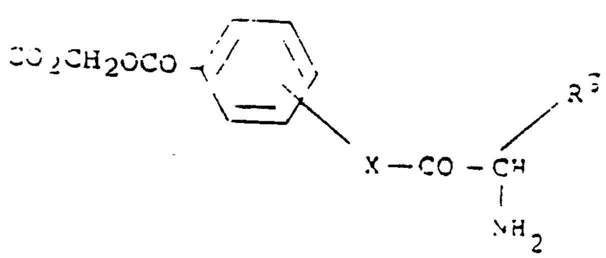

(iv)

wherein $R^a$ is hydrogen, $C_{1-6}$ alkyl, $C_{3-7}$ cycloalkyl, or phenyl, $R^b$ is $C_{1-6}$ alkyl, $C_{1-6}$ alkoxy, phenyl, benzyl, $C_{3-7}$ cycloalkyl, $C_{1-6}$ alkyl-$C_{3-7}$ cycloalkyl, 1-amino $C_{1-6}$ alkyl, or 1-($C_{1-6}$ alkyl)amino $C_{1-6}$ alkyl; or $R^a$ and $R^b$ together form a 1,2-phenylene group optionally substituted by one or two methoxy groups; $R^c$ represents $C_{1-6}$ alkylene optionally substituted with a methyl or ethyl group and $R^d$ and $R^e$ independently represent $C_{1-6}$ alkyl; $R^f$ represents $C_{1-6}$ alkyl; $R^g$ represents hydrogen or phenyl optionally substituted by up to three groups selected from halogen, $C_{1-6}$ alkyl, or $C_{1-6}$ alkoxy; and X is (preferably o) oxygen or (preferably o or p)NH.

Examples of suitable in vivo hydrolysable ester groups include, for example, acyloxyalkyl groups such as acetoxymethyl, pivaloyloxymethyl, α-acetoxyethyl, α-pivaloyloxyethyl, 1-(cyclohexylcarbonyloxy)prop-1-yl, and (1-aminoethyl)carbonyloxymethyl; alkoxycarbonyloxyalkyl groups, such as ethoxycarbonyloxymethyl and α-ethoxycarbonyloxyethyl; dialkylaminoalkyl especially di-loweralkylamino alkyl groups such as dimethylaminomethyl, dimethylaminoethyl, diethylaminomethyl or diethylaminoethyl; lactone groups such as phthalidyl and dimethoxyphthalidyl; and esters linked to a second β-lactam antibiotic or to a β-lactamase inhibitor.

A further suitable pharmaceutically acceptable in vivo hydrolysable ester group is that of the formula:

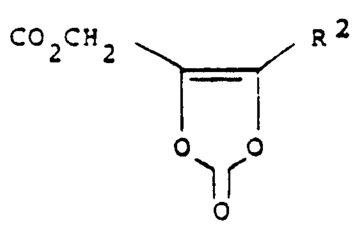

wherein $R^2$ is hydrogen, $C_{1-6}$ alkyl or phenyl.

Suitable pharmaceutically acceptable salts of the carboxy group of the compound of formula (I) include

metal salts, eg aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine, cycloalkylamines such as dicyclohexylamine, or with procaine, dibenzylamine, N,N-dibenzylethylenediamine, 1-ephenamine, N-ethylpiperidine, N-benzyl-β-phenethylamine, dehydroabietylamine, N,N′-bisdehydroabietylamine, ethylenediamine, or bases of the pyridine type such as pyridine, collidine or quinoline, or other amines which have been used to form salts with known penicillins and cephalosporins.

Some of the compounds of this invention may be crystallised or recrystallised from solvents such as methanol. In such cases solvates may be formed. This invention includes within its scope stoichiometric solvates including hydrates as well as compounds containing variable amounts of water that may be produced by processes such as lyophilisation.

Since the compounds of the formula (I) and their salts and in-vivo hydrolysable esters are intended for use in pharmaceutical compositions it will readily be understood that they are each provided in substantially pure form, for example at least 60% pure, more suitably at least 75% pure and preferably at least 85%, especially at least 95% pure (% are on a weight for weight basis). Impure preparations of the compounds of the formula (I) and their salts may be used for preparing the more pure forms used in the pharmaceutical compositions; these less pure preparations of the compounds of formula (I) and their salts should contain at least 1%, more suitably at least 5% and preferably from 10 to 49% of a compound of the formula (I) or salt thereof.

Compounds of the present invention may exist as either syn or anti isomers, or may exist as mixtures of syn and anti isomers containing at least 75% of one such isomer, or preferably at least 90% of one such isomer.

Herein the terms syn and anti refer to the configuration of the group OR with respect to the carboxamido group, the syn-configuration (sometimes called the Z-configuration) being denoted thus:

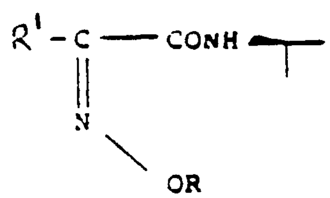

and the anti configuration (sometimes called the E-configuration) being denoted thus:

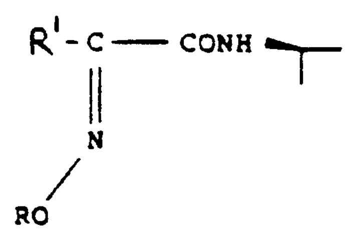

Preferred compounds of the present invention are the syn-isomers of the formula (II):

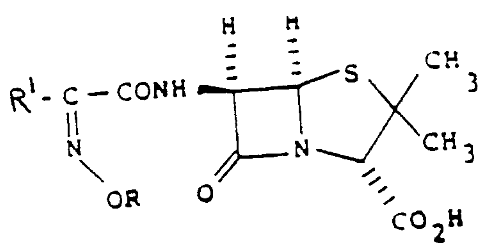

(II)

wherein R and $R^1$ are as hereinbefore defined.

A particularly preferred compound within the present invention is the compound of formula (II) or a pharmaceutically acceptable salt or in vivo hydrolysable ester thereof wherein R is 1-methylcyclopentyl or 1-methylcyclohexyl, and $R^1$ is 2-fluorophenyl:

6β-[Z-2-(2-Fluorophenyl)-2-(1-methylcyclopentyloxyimino)acetamido]penicillanic acid, and

6β-[Z-2-(2-fluorophenyl)-2-(1-methylcyclohexyloxyimino)acetamido]penicillanic acid.

The compounds of formula (I) may be prepared by treating a compound of formula (III) or salt thereof:

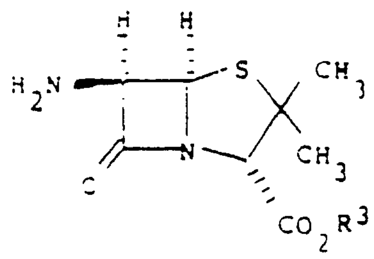

(III)

wherein the amino group is optionally substituted with a group which permits acylation to take place, and $R^3$ is hydrogen or a readily removable carboxyl blocking group; with an acylating agent derived from the acid of formula (IV):

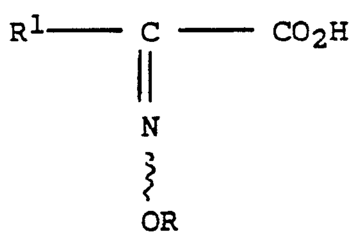

(IV)

wherein R and $R^1$ are as defined with respect to formula (I) Any of the following reactions in any appropriate sequence may then be carried out:-

(i) removal of any carboxyl blocking group $R^3$;

(ii) formation of a pharmaceutically acceptable salt;

(iii) conversion of a carboxyl group into an ester function such as an in vivo hydrolysable ester.

Suitable groups which permit acylation to take place and which are optionally present on the amino group of the starting material of formula (III) include N-silyl, N-stannyl and N-phosphorus groups, for example trialkylsilyl groups such as trimethylsilyl, trialkyltin groups such as tri-n-butyltin, groups of formula $-PR^aR^b$ wherein $R^a$ is an alkyl, haloalkyl, aryl, aralkyl, alkoxy, haloalkoxy, aryloxy, aralkoxy or dialkylamino group, $R^b$ is the same as $R^a$ or is halogen or $R^a$ and $R^b$ together form a ring; suitable such phosphorus groups being $-P(OC_2H_5)_2$, $-P(C_2H_5)_2$,

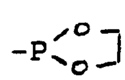

and

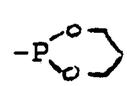.

Suitable carboxyl-blocking derivatives for the group $CO_2R^3$ in formula (III) include salts and ester derivatives of the carboxylic acid. The derivative is preferably one which may readily be cleaved at a later stage of the reaction. Suitable salts include metal salts, such as those with sodium, potassium and lithium, and tertiary amine salts, such as those with trilower-alkylamines, N-ethylpiperidine, 2.6-lutidine, pyridine, N-methylpyrrolidine, dimethylpiperazine. A preferred salt is with triethylamine.

Suitable ester-forming carboxyl-blocking groups are those which may be removed under conventional conditions. Such groups for $R^3$ include benzyl, p-methoxybenzyl, 2,4,6-trimethylbenzyl, 3,5-di-t-butyl-4-hydroxy-benzyl, benzoylmethyl, p-nitrobenzyl, 4-pyridylmethyl, 2,2,2-trichloroethyl, 2,2,2-tribromoethyl, t-butyl, t-amyl, diphenylmethyl (benzhydryl), triphenylmethyl, adamantyl,2-benzyloxyphenyl, 4-methylthiophenyl, tetrahydrofuran-2-yl, tetrahydropyran-2-yl, pentachlorophenyl, p-toluene-sulphonylethyl, methoxymethyl, a silyl, stannyl or phosphorus-containing group, such as described above, an oxime radical of formula -N=CHR$^4$ where $R^4$ is aryl or heterocyclic, or an in vivo hydrolysable ester radical such as defined above.

The carboxyl group may be regenerated from any of the above esters by usual methods appropriate to the particular $R^3$ group, for example, acid - and base -catalysed hydrolysis, or by enzymically - catalysed hydrolysis, or by hydrogenation under conditions wherein other parts of the molecule are unaffected.

A reactive N-acylating derivative of the acid of formula (IV) is employed in the above process. The choice of reactive derivative will be influenced by the chemical nature of the group R.

Suitable N-acylating derivatives of the acid (IV) include acid (IV) halides, preferably the acid chloride or bromide. Acylation with an acid halide may be effected in the presence of an acid binding agent, for example a tertiary amine (such as triethylamine or dimethylaniline), an inorganic base (such as calcium carbonate or sodium bicarbonate), molecular sieves (such as type 4 Angstroms) or an oxirane, which binds hydrogen halide liberated in the acylation reaction. The oxirane is preferably a ($C_{1-6}$)- 1,2-alkylene oxide - such as ethylene oxide or propylene oxide. The acylation reaction using an acid halide may be carried out at a temperature in the range -50°C to +50°C, preferably -20°C to +20°C, in aqueous or non-aqueous media such as aqueous acetone, aqueous tetrahydrofuran, ethyl acetate, dimethylacetamide, dimethylformamide (DMF), acetonitrile, dichloromethane, 1,2-dichloroethane, or mixtures thereof. Alternatively, the reaction may be carried out in an unstable emulsion of water-immiscible solvent, especially an aliphatic ester or ketone, such as methyl isobutyl ketone or butyl acetate.

The acid halide may be prepared by reacting the acid (IV) with a halogenating (eg chlorinating or brominating) agent such as phosphorus pentachloride, thionyl chloride or oxalyl chloride.

Alternatively, the N-acylating derivative of the acid (IV) may be a symmetrical or mixed anhydride. Suitable mixed anhydrides are alkoxyformic anhydrides, or anhydrides with, for example, carbonic acid monoesters, trimethyl acetic acid, thioacetic acid, diphenylacetic acid, benzoic acid, phosphorus acids (such as phosphoric or phosphorous acids) or aliphatic or aromatic sulphonic acids (such as methanesulphonic acid and p-toluenesulphonic acid respectively). When a symmetrical anhydride is employed, the acylation reaction may be carried out in the presence of an organic base such as 2,6-lutidine as catalyst.

When a mixed anhydride is employed the N-acylating derivative is preferably prepared in the presence of an organic base such as triethylamine and/or N,N-diisopropylethylamine in a suitable solvent such as DMF at between -50°C and room temperature. Alternatively, the N-acylating derivative may be prepared from an alkali metal salt of the acid of formula (IV), such as the sodium salt, in a suitable solvent such as DMF at between -50°C and room temperature. The N-acylating derivative of the acid of formula (IV) so derived may then be reacted with a compound of formula (III). The acylation reaction may conveniently be carried out at -50°C to +50°C, preferably not more than 0°C, in a suitable solvent such as water, acetonitrile or DMF. The reaction may be carried out in the presence of a suitable base such as triethylamine or sodium hydrogen carbonate.

A further method of forming the N-acylating derivative of the acid of formula (IV) is to treat the acid of formula (IV) with a solution or suspension preformed by addition of a carbonyl halide, preferably oxalyl chloride, or a phosphoryl halide such as phosphorus oxychloride, to a halogenated hydrocarbon solvent, preferably dichloromethane, containing a lower acyl tertiary amide, preferably N,N-dimethylformamide. The N-acylating derivative of the acid of formula (IV) so derived may then be caused to react with a compound of formula (III). The acylation reaction may conveniently be carried out at -40° to +30°C, if desired in the presence of an acid binding agent such as triethylamine. A catalyst such as 4-dimethylaminopyridine may optionally also be added.

Other suitable acylating agents derived from the acid of formula (IV) are thioesters of formula (V)

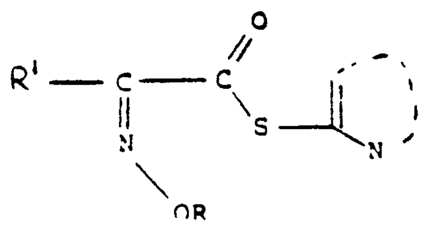

(V)

wherein R and $R^1$ are as hereinbefore defined and represents a 5- or 6-membered heterocyclic ring, which may contain, in addition to the nitrogen atom, one or two further heteroatoms, selected from oxygen, nitrogen and sulphur and which may be substituted or fused to a benzene ring which may itself be substituted.

Particular acylating agents derived from the acid of formula (IV) are the thio esters (Va) or (Vb)

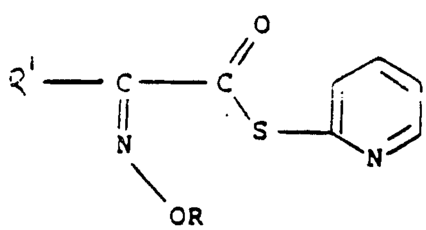

(Va)

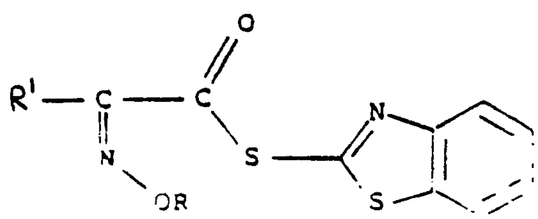

(Vb)

wherein R and $R^1$ are as hereinbefore defined.

Compounds of the formulae (Va) and (Vb) may be prepared by treatment of the acid (V) with 2,2′-dipyridyldisulphide or 2,2′- dibenzothiazolyldisulphide respectively, in the presence of triphenylphosphine, analogously to the routes described in EP-A-0037380.

Other suitable N-acylating derivatives of acid (IV) include the acid azide; the activated esters derived from cyanomethanol; p-nitrophenol; 2,4-dinitrophenol; thiophenol; halophenols, including pentachlorophenol; monomethoxyphenol; N-hydroxy succinimide; N-hydroxybenzotriazole or 8-hydroxyquinoline; or include amides such as N-acylsaccharins, N-acylthiazolidin-2-thione or N-acylphthalimides; or an alkylidene iminoester prepared by reaction of the acid (IV) with an oxime.

Compounds of formula (IV) may be prepared by routes analogous to those disclosed in GB-A-1,399,087, GB-A-2 025 398 and EP-A-0 210 815.

In particular, compounds of formula (IV) may be prepared as shown in Scheme I or II:

Scheme I

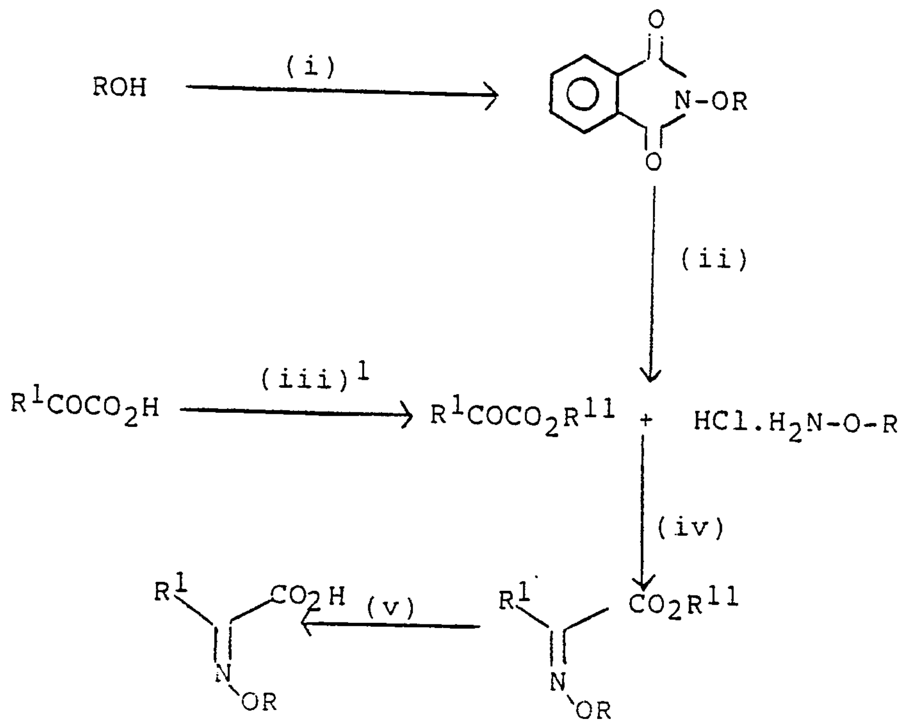


Reagents:

(i) N-Hydroxyphthalimide, 4A molecular sieves; $BF_3.Et_2O$, MDC, Reflux
(ii) $H_2NNH_2.H_2O$, EtOH, Reflux; HCl.
(iii) $Me_3SiCl$, $R^{11}OH$, 20h.$R^{11}$ is methyl or ethyl.
(iv) $R^{11}OH$, RT, 20h. E/Z-isomer separation. (v) NaOH, $R^{11}OH$, RT.

Reference:

1. T.H. Chan and M.A. Brook, Synthesis, 1983, 201.

Scheme II

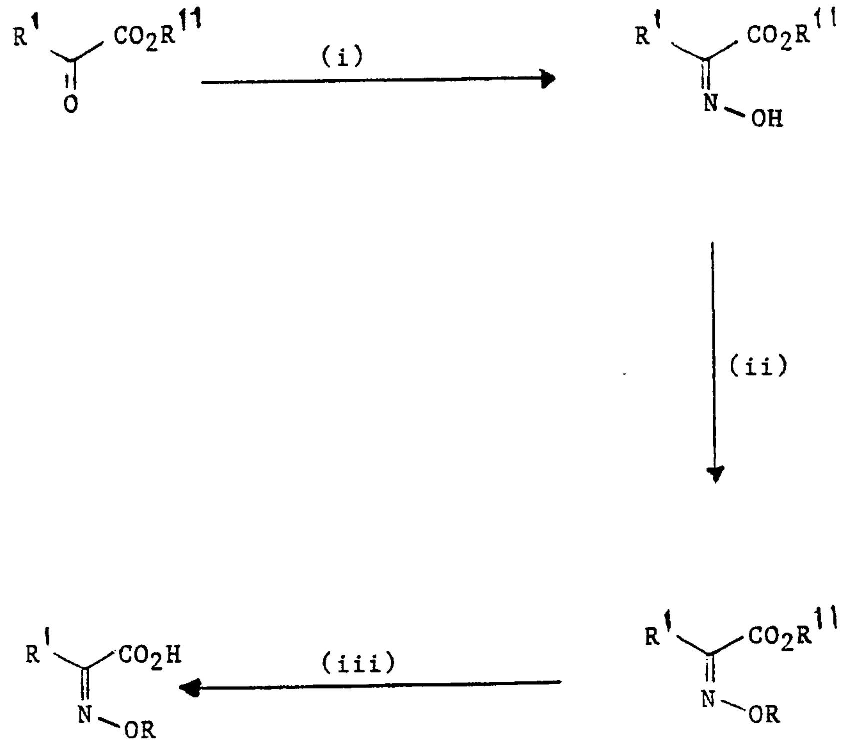


Reagents:

(i) Hydroxylamine hydrochloride, $R^{11}OH$, $H_2O$. E/Z-isomer separation.
(ii) ROH, $BF_3.Et_2O$, molecular sieves, MDC, reflux, inert atmosphere. E/Z-isomer separation.
(iii) NaOH, $R^{11}$ OH, RT.

The present invention also provides a pharmaceutical composition which comprises a compound of formula (I) or a pharmaceutically acceptable salt or in vivo hydrolysable ester thereof and a pharmaceutically acceptable carrier. The compositions of the invention include those in a form adapted for oral, topical or parenteral use and may be used for the treatment of the infection in mammals including humans.

The antibiotic compounds according to the invention may be formulated for administration in any convenient way for use in human or veterinary medicine, by analogy with other antibiotics, and the invention therefore includes within its scope a pharmaceutical composition comprising a compound of formula (I) above together with a pharmaceutical carrier or excipient.

The composition may be formulated for administration by any route, such as oral, topical or parenteral. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrollidone: fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The tablets may be

coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the composition comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (I) is administered in the above-mentioned dosage range.

Compounds of the present invention are characterised by stability to β-lactamase producing organisms.

The compound of the invention of formula (I) may therefore be used as the sole therapeutic agent in compositions of the invention or may be used in combination with other antibiotics or with a β-lactamase inhibitor.

Advantageously the compositions also comprise a compound of formula (VI) or a pharmaceutically acceptable salt or ester thereof:

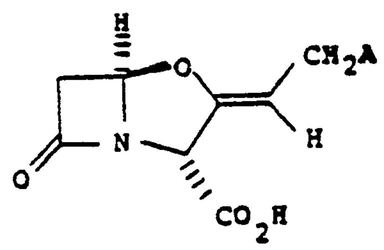

(VI)

wherein A is hydroxyl; substituted hydroxyl; thiol; a group of formula $SO_2R_5$ wherein $R_5$ is $C_{1-6}$ alkyl; substituted thiol; amino; mono- or di-hydrocarbyl substituted amino; mono- or di-acylamino; an optionally substituted triazolyl group; or an optionally substituted tetrazolyl group as described in EP O 053 893.

A further advantageous composition comprises an antibiotic compound according to the invention and a pharmaceutically acceptable carrier or excipient together with a β-lactamase inhibitor of formula (VII) or a pharmaceutically acceptable salt or in vivo hydrolysable ester thereof:

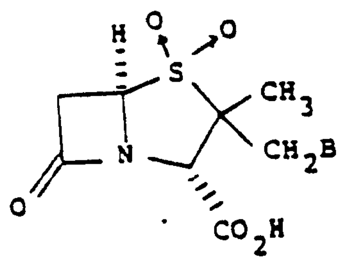

(VII)

wherein B is hydrogen, halogen or a group of formula:

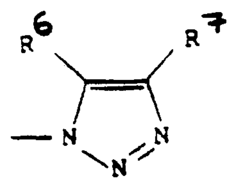

in which $R^6$ and $R^7$ are the same or different and each is hydrogen, $C_{1-6}$ alkoxycarbonyl, or carboxy or a pharmaceutically acceptable salt thereof.

Further suitable β-lactamase inhibitors include 6-alkylidene penem of formula (VIII) below:

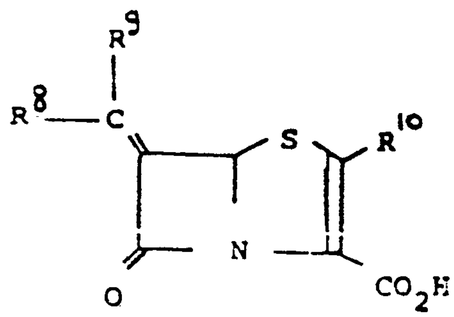

(VIII)

or a pharmaceutically acceptable salt or in vivo hydrolysable ester thereof, wherein $R^8$ and $R^9$ are the same or different and each represents hydrogen, or a $C_{1-10}$ hydrocarbon or heterocyclic group optionally substituted with a functional group; and $R^{10}$ represents hydrogen or a group of formula $R^a$ or $-SR^a$ where $R^a$ is an optionally substituted $C_{1-10}$ hydrocarbon or heterocyclic group, as described in European Patent Application No. 81301683.9 (Publication Number 0 041 768).

Other suitable β-lactamase inhibitors include 6β-bromopenicillanic acid and salts and in vivo hydrolysable esters thereof and 6β-iodopenicillanic acid and salts and in vivo hydrolysable esters thereof.

Such compositions of this invention comprising a β-lactamase inhibitor are formulated in conventional manner.

The present invention also includes a method of treating bacterial infections in humans and animals which comprises the administration of a therapeutically effective amount of an antibiotic compound of this invention.

Antibiotic compounds of the present invention are active against a range of bacteria, in particular they are useful for treatment of respiratory tract and urinary tract infections in humans and mastitis in cattle.

The antibiotic compounds of the present invention are active against organisms including H.influenzae, in particular Q1 and NEMC 1;S.aureus such as Oxford, Russell and MB 9; S.pyogenes such as CN10; S.agalactiae such as 2798; and S.pneumoniae such as PU7 and 1761.

The following Examples illustrate the preparation of the compounds of the present invention.

Example 1

Sodium 6β-[Z-2-(2-Fluorophenyl)-2-(1-methylcyclopentyloxyimino)acetamido]penicillanate.-

a) N-(1-methylcyclopentyloxy)phthalimide.-

N-Hydroxyphthalimide (8.15g), 1-methylcyclopentanol (5.00g) and 4A molecular sieves (25g) were stirred in dry dichloromethane (250ml) under argon for 23 min at room temperature. Boron trifluoride etherate (5.00ml) was added and the mixture heated to reflux and stirred for $2^{3}/_{4}$h. The mixture was allowed to cool and filtered. The filtrate was washed succesively with water (100ml), 1M sodium hydrogen carbonate solution (2x100ml), water (100ml) and saturated sodium chloride solution (100ml), dried over magnesium sulphate and concentrated in vacuo. The concentrate was crystallized from hexane, filtered and the product washed with chilled hexane (10ml) to give the title compound as a white solid (2.64g), m.p. 83-84.5° C; $\nu_{max}$ (KBr) 1785, 1740, 1610, 1354, 966, 878, 706cm$^{-1}$; $\delta_H$ ($CDCl_3$) 1.2-2.5 (11H,m, incorporating singlet 1.47), 7.6-8.0 (4H,m); m/z CI ($NH_3$ gas) $MNH_4^+$, 263 and $MH^+$, 246.

b) 1-Methylcyclopentyloxyamine hydrochloride.-

Hydrazine hydrate (0.65ml) was added to N-(1-methylcyclopentyloxy)phthalimide (2.48g) in ethanol (40ml) at reflux temperature. The mixture was stirred at reflux for 1h 10 min, then allowed to cool and poured into 3% sodium carbonate solution (150ml). The solution was extracted with diethyl ether (6x75ml), the combined organic phases were washed with saturated sodium chloride solution (40ml), dried over magnesium sulphate and concentrated in vacuo. The concentrate was dissolved in hexane (150ml) and hydrogen chloride gas was bubbled through the hexane solution for 8 min (ice bath cooling at the beginning). The mixture was concentrated in vacuo. and crystallized from hexane. The product was filtered and washed with chilled hexane to give the title amine hydrochloride (1.19g, 78%), (Found: C, 47.64; H, 9.47; N, 9.31; Cl, 23.54%. $C_{16}H_{14}NOCl$ requires C, 47.53; H, 9.31; N, 9.24; Cl, 23.38%.) $\nu_{max}$ (KBr) 1558, 1454, 1384, 1339, 1199, 994cm$^{-1}$; $\delta_H$ ($(CD_3)_2SO$) 1.1-2.4 (m, incorporating singlet 1.45).

(c) Methyl 2-Fluoro-α-oxo-benzeneacetate.-

Chlorotrimethylsilane (10.4ml) was added to 2-fluoro-α-oxo-benzeneacetic acid (6.24g) in dry methanol (50ml) and stirred at room temperature under an argon atmosphere for 20h. The mixture was concentrated in vacuo, and the title compound obtained by flash chromatography (Kieselgel 60 [Merck: Art.7729 (finer than 230 mesh ASTM)], hexane-ethyl acetate (9:1)) (5.89g, 87%), $\nu_{max}$ (film) 1750, 1690, 1613, 1583, 1485, 1010cm$^{-1}$; $\delta_H$ ($CDCl_3$) 3.93 (3H,s), 6.9-8.1 (4H,m).

(d) Methyl 2-Fluoro-α-(Z-1-methylcyclopentyloxyimino)benzeneacetate.-

1-Methylcyclopentyloxyamine hydrochloride (1.90g) was added to methyl 2-fluoro-α-oxo-benzeneacetate (2.29g) in methanol (12ml) and the mixture stirred for 20h at room temperature. The reaction mixture was partitioned between ethyl acetate (40ml) and water (20ml). The aqueous phase was extracted with ethyl acetate (20ml) and the combined organic phases were washed succesively with water (20ml) and saturated sodium chloride solution (20ml), dried over magnesium sulphate and concentrated in vacuo. The crude product was chromatographed [Kieselgel 60, hexane-ethyl acetate (19:1)] to give the title compound - (0.82g). Further elution of the column afforded a mixture of E- and Z-oxime isomers, followed by the pure E-isomer of the title compound (0.82g). The mixture was re-chromatographed to provide an additional amount of the Z-isomer. The total yield of the title compound (Z-isomer) was 0.88g. (Found: $M^+$, 279.1277. $C_{15}H_{18}NO_3F$ requires M 279.1271); $\nu_{max}$ (film) 1745, 1615, 1598, 1490, 1265, 965cm$^{-1}$; $\delta_H$ ($CDCl_3$) 1.2-2.3 (11H,m, incorporating singlet 1.47), 3.86 (3H,s), 6.8-7.5 (3H,m), 7.6-7.9 (1H,m); m/z (EI) $M^+$, 279.

(e) 2-Fluoro-α-(Z-1-methylcyclopentyloxyimino)benzeneacetic acid.-

1M sodium hydroxide solution (6.20ml) was added to methyl 2-fluoro-α-(Z-1-methylcyclopentyloxyimino)benzeneacetate (0.87g) in methanol (10ml) and the mixture stirred for 67 h at room temperature. The

reaction mixture was partitioned between ethyl acetate (25ml) and water (20ml) and acidified to pH 2 with 1M hydrochloric acid. The aqueous phase was extracted with ethyl acetate (10ml) and the combined organic phases washed with water (10ml) and with saturated sodium chloride solution (10ml), dried over magnesium sulphate and concentrated in vacuo to give the crude product. Recrystallization (hexane) gave the title compound (0.51g, 62%), m.p. 90-92.5° C. (Found: C, 63.06; H, 6.07; N, 5.27%; $M^+$, 265.1121. $C_{14}H_{16}NO_3F$ requires C, 63.43; H. 6.08; N, 5.28%; M, 265.1114.) $\nu_{max}$ (KBr) 2972, 1718, 1612, 1596, 1268, 968cm$^{-1}$; $\delta$($CDCl_3$) 1.1-2.5 (11H,m, incorporating singlet 1.51), 6.9-8.0 (4H,m); m/z (EI) $M^+$, 265.

(f) Sodium 6$\beta$-[Z-2-(2-Fluorophenyl)-2-(1-methylcyclopentyloxyimino)acetamido]penicillanate.-

Oxalyl chloride (0.315ml) was added to a solution of N,N-dimethylformamide (0.277ml) in dry dichloromethane (10ml) at -20° C and stirred under an atmosphere of argon for 12 min. 2-Fluoro-$\alpha$-(Z-1-methylcyclopentyloxyimino)benzeneacetic acid (0.633g) in dry dichloromethane (5ml) was added to the mixture and stirred at -18° C for 14 min. A mixture of triethylammonium 6-aminopenicillanate (1.513g) and triethylamine (0.664ml) in dry dichloromethane (5ml) was then added, and after 10 min between -11° C and -17° C, the cooling bath was removed and the reaction stirred for 2h 20 min. The reaction mixture was washed with dilute hydrochloric acid (0.25M, 30ml). The aqueous phase was extracted with dichloromethane (25ml) and the combined organic phases washed successively with water (25ml) and saturated sodium chloride solution (25ml), dried over magnesium sulphate and concentrated in vacuo to a pale foam.

The crude product was dissolved in dilute sodium hydrogen carbonate solution, and was chromatographed using HP20SS resin employing gradient elution with acetone-water mixtures (0:1)-(1:3). The fractions containing the product (hplc analysis) were lyophilized to give the title compound as a white solid (0.76g, 66%). $\nu_{max}$ (KBr) 1773, 1674, 1611, 1512, 1451. 1401. 952cm$^{-1}$; $\delta_H$ [250MHz, $(CD_3)_2SO$] 1.0-1.85 (15H,m, incorporating singlets at 1.42, 1.46 and 1.55), 1.85-2.2 (2H,m), 3.85 (1H,s,3-H), 5.42 (1H,d,J 4Hz,5-H), 5.51 (1H,dd,J 4Hz and 8Hz,6-H), 7.1-7.4 (2H,m), 7.4-7.54 (1H,m), 7.54-7.7 (1H,m), 9.10 (1H,d,J 8Hz,$D_2O$ exch.,CONH); m/z (FAB) $MH^+$, 486.

Alternatively, the crude product (3.05g) was dissolved in a 1:1 mixture of t-butenol and water (90ml), and the pH of the chilled solution adjusted to 7 with 0.1M sodium hydroxide solution. The solution was partially concentrated in vacuo. then freeze dried to give the title compound (2.84g).

## Example 2

Sodium 6$\beta$-[Z-2-(2-Fluorophenyl)-2-(1-methylcyclohexyloxyimino)acetamido]penicillanate.-

(a) N-(1-Methylcyclohexyloxy)phthalimide.-

The title compound was prepared in a manner similar to that described for Example 1(a). Reaction of N-hydroxyphthalimide (8.15g), 1-methylcyclohexanol (6.21ml) and boron trifluoride etherate (4.95ml) in dry dichloromethane in the presence of 4A molecular sieves gave the title compound (3.40g), m.p. 98-99° C; $\nu_{max}$ (KBr) 1785, 1742, 1611, 1465, 879, 709cm$^{-1}$; $\delta_H$ ($CDCl_3$) 1.1-2.3 (13H,m, incorporating singlet 1.33), 7.7-8.2 (4H,m); m/z CI ($NH_3$ gas) $MNH_4^+$, 277 and $MH^+$, 260.

(b) 1-Methylcyclohexyloxyamine hydrochloride.-

The title compound was prepared in a manner similar to that described in Example 1(b). Reaction of hydrazine hydrate (0.79ml) with N-(1-methylcyclohexyl oxy)phthalimide (3.15g) gave the title compound - (1.40g, 70%), (Found: C, 50.79; H, 9.63; N, 8.58; Cl, 21.65%. $C_7H_{16}NOCl$ requires C, 50.75; H, 9.74; N, 8.46; Cl, 21.40%). $\nu_{max}$ (KBr) 2932 (br), 2656 (br), 1936 (br), 1591, 1561, 1537, 1449, 984, 801cm$^{-1}$; $\delta_H$ [-$(CD_3)_2SO$] 1.0-2.2 (13H, incorporating singlet 1.33), 9.7-11.7 (br).

(c) Methyl 2-Fluoro-$\alpha$-(Z-1-methylcyclohexyloxyimino)benzeneacetate.-

The title compound was prepared in a manner similar to that described for Example 1(d). Reaction of methyl 2-fluoro-α-oxo-benzeneacetate (1.32g) with 1-methylcyclohexyloxyamine hydrochloride (1.20g) followed by chromatography [Kieselgel 60; elution with hexaneethyl acetate (19:1)] gave the title compound (0.63g, 30%) as a colourless oil, $\nu_{max}$ (film) 1745, 1615, 1600, 1490, 1265, 1215, 970cm$^{-1}$; $\delta_H$ ($CDCl_3$) 1.0-2.4 (m, incorporating singlet 1.33), 3.87 (s), 6.9-7.5 (m), 7.6-7.9 (m); m/z CI ($NH_3$ gas) $MH^+$, 294.

(d) 2-Fluoro-α-(Z-1-methylcyclohexyloxyimino)benzeneacetic acid.-

The title compound was prepared in a manner similar to that described for Example 1(e). Treatment of methyl 2-fluoro-α-(Z-1-methylcyclohexyloxyimino)benzeneacetate (0.43g) with 1M sodium hydroxide solution (2.94ml) gave the title compound (0.31g, 76%) after recrystallization from hexane, m.p. 87-88°C (Found; C, 64.44; H, 6.46; N, 4.96%. $C_{15}H_{18}NO_3F$ requires C, 64.50; H, 6.50; N, 5.01%). $\nu_{max}$ (KBr) 1725, 1708, 1611, 1595, 1487, 1269, 974cm$^{-1}$; $\delta_H$ ($CDCl_3$) 1.1-2.5 (13H,m, incorporating singlet 1.37), 6.9-7.8 (4H,m), 10.0-10.4 (1H,br); m/z CI ($NH_3$ gas) $MH^+$, 280.

(e) Sodium 6β-[Z-2-(2-Fluorophenyl)-2-(1-methylcyclohexyloxyimino)acetamido]penicillanate.-

The title compound was prepared in a manner similar to that described for Example 1(f). Reaction between 2-fluoro-α-(Z-1-methylcyclohexyloxyimino)benzeneacetic acid (0.335g), oxalyl chloride (0.158ml), N,N-dimethylformamide (0.139ml), triethylammonium 6-aminopenicillanate (0.760g) and triethylamine (0.334ml), followed by chromatography [HP20SS, acetone-water (0:1)-(3:7)] and lyophilization gave the title compound (0.38g, 63%), $\nu_{max}$ (KBr) 1774, 1670, 1611, 1512, 1449, 1401, 963, 953cm$^{-1}$; $\delta_H$ [250MHz, $(CD_3)_2SO$] 1.1-1.75 (17H,m, incorporating singlets 1.28, 1.46 and 1.55), 1.75-2.0 (2H,m), 3.86 (1H,s,3-H), 5.43 (1H,d,J 4Hz,5-H), 5.55 (1H,dd,J 4 and 8Hz,6-H), 7.15-7.38 (2H,m), 7.38-7.54 (1H,m), 7.54-7.66 (1H,m), 9.12 (1H,d,J 8Hz,$D_2O$ exch., CONH); m/z (FAB) $MH^+$, 500.

Example 3

Sodium 6β-[Z-2-(2-Fluorophenyl)-2-(1-methylcycloheptyloxyimino)acetamido]penicillanate.-

(a) N-(1-Methylcycloheptyloxy)phthalimide.-

The title compound was prepared in a manner similar to that described for Example 1(a). Reaction of N-hydroxyphthalimide (7.63g), 1-methylcycloheptanol (6.90ml) and boron trifluoride etherate (4.63ml) in dry dichloromethane (250ml) in the presence of 3A molecular sieves (25g) gave the title compound (3.03g) after chromatography [Kieselgel, hexane-ethyl acetate (19:1)-(4:1)], m.p. 76-78.5°C (Found: C, 70.58; H, 7.04; N, 5.11%. $C_{16}H_{19}NO_3$ requires C, 70.31; H, 7.01; N, 5.12%); $\nu_{max}$ (KBr) 1787, 1745, 1611, 1373, 1350, 967, 878, 711cm$^{-1}$; $\delta_H$ ($CDCl_3$) 1.1-2.6 (15H,m, incorporating singlet 1.39), 7.7-8.3 (4H,m); m/z CI ($NH_3$ gas) $MNH_4^+$, 291 and $MH^+$, 274.

(b) 1-Methylcycloheptyloxyamine hydrochloride

The title compound was prepared in a manner similar to that described for Example 1(b). Reaction of hydrazine hydrate (0.47ml) with (1-methylcycloheptyloxy)phthalimide (1.98g) in ethanol (25ml) gave the title compound (0.48g, 37%), $\nu_{max}$ (KBr) 1576,1559 1479, 1386, 1194, 1172, 1000cm$^{-1}$; $\delta_H$ [$(CD_3)_2SO$] 1.0-2.2 (m, incorporating singlet 1.33), 9.0-11.5 (br).

(c) Methyl 2-Fluoro-α-(Z-1-methylcycloheptyloxyimino)benzeneacetate.-

1-Methylcycloheptyloxyamine hydrochloride (0.33g) was added to methyl 2-fluoro-α-oxo-benzeneacetate (0.34g) in methanol (3ml) and the reaction mixture stirred at room temperature for 26h. The

reaction mixture was partitioned between ethyl acetate and water and the aqueous phase was extracted with ethyl acetate. The combined organic phases were washed with water (10ml) and with saturated sodium chloride solution (10ml), dried over magnesium sulphate and concentrated in vacuo. 'Flash chromatography' of the residue [Kieselgel, ethyl acetate-hexane (1:9)] gave methyl 2-fluoro-α-(1-methylcycloheptyloxyimino)-benzeneacetate (0.50g) as a mixture of Z- and E-isomers. 1M sodium hydroxide solution (2.12ml) was added to the mixture of isomers (0.49g) in methanol (8ml) and stirred for 4h.

The reaction mixture was partitioned between ethyl acetate (20ml) and water (10ml) and the aqueous phase extracted with ethyl acetate. The combined organic phases were washed successively with water (10ml) and saturated sodium chloride solution (10ml), were dried over magnesium sulphate and concentrated in vacuo. 'Flash chromatography' of the residue [Kieselgel; ethyl acetate-hexane (1:9)] gave the title compound (0.124g), (Found: $M^+$, 307.1556. $C_{17}H_{22}NO_3F$ requires M, 307.1584.); $\nu_{max}$ (film), 1750, 1615, 1595, 1490, 1455, 1265, 970$cm^{-1}$; $\delta_H$ ($CDCl_3$) 1.1-2.6 (15H,m, incorporating singlet 1.36), 3.87 (s), 6.8-8.1 (4H,m); m/z (EI) $M^+$, 307.

(d) 2-Fluoro-α-(Z-1-methylcycloheptyloxyimino) benzeneacetic acid.-

1M sodium hydroxide solution (0.81ml) was added to methyl 2-fluoro-α-(Z-1-methylcycloheptyloxyimino) benzeneacetate (0.12g) in methanol (3ml) and stirred at room temperature for 27h. Additional 1M sodium hydroxide solution (0.16ml) was added and the mixture was stirred overnight. The reaction mixture was partitioned between ethyl acetate and water and acidified with 1M hydrochloric acid. The aqueous phase was extracted with ethyl acetate and the combined organic phases were washed with water and with saturated sodium chloride solution, dried over magnesium sulphate and concentrated in vacuo to give the title compound (0.08g, 70%), (Found: $M^+$, 293.1407. $C_{16}H_{20}NO_3F$ requires M, 293.1427.) $\nu_{max}$ (KBr) 1723, 1612, 1596, 1486, 1455, 1420, 1268, 968$cm^{-1}$; $\delta_H$ ($CDCl_3$) 1.0-2.6 (15H,m, incorporating singlet 1.38) 6.9-7.8 (4H,m), 9.5-9.9 (1H,s); m/z (EI) $M^+$, 293.

(e) Sodium 6β-[Z-2-(2-Fluorophenyl)-2-(1-methylcycloheptyloxyimino)acetamido]penicillanate.-

The title compound was prepared in a manner similar to that described for Example 1(f). Reaction between 2-fluoro-α-(Z-1-methylcycloheptyloxyimino)benzeneacetic acid (0.08g), oxalyl chloride (36μl), N,N-dimethyl formamide (31μl), triethylammonium 6-aminopenicillanate (0.171g) and triethylamine (75μl), then HP20SS chromatography, eluting with acetone-water mixtures (0:1)-(3:7) provided the title compound as a lyophilized solid (0.112g, 80%), $\nu_{max}$ (KBr) 1774, 1671, 1609, 1509, 1401, 1319, 951$cm^{-1}$; $\delta_H$ [250MHz, $(CD_3)_2SO$] 1.2-1.8 (m, incorporating singlets 1.70, 1.46 and 1.54), 1.9-2.15 (2H,m), 3.84 (1H,s,3-H), 5.42 (1H,d,J 4Hz 5-H), 5.51 (1H,dd,J 4 and 8Hz, 6-H), 7.15-7.35 (2H,m), 7.40-7.53 (1H,m), 7.53-7.63 (1H,m), 9.05 (1H,d,J 8Hz $D_2O$ exch., CONH); m/z (FAB) $MNa^+$, 536.

## Example 4

### Sodium 6β-[Z-2-(1-Methylcyclopentyloxyimino)-2-phenylacetamido]penicillanate.-

(a) Ethyl α-(Z-1-Methylcyclopentyloxyimino)benzeneacetate.-

The title compound was prepared in a similar manner to that described for Example 3(c). Reaction between ethyl α-oxo-benzeneacetate (0.445g) and 1-methylcyclopentyloxyamine hydrochloride (0.38g) in ethanol (2.5ml) followed by flash chromatography [Kieselgel, ethyl acetate-hexane (0:1)-(1:19)] gave ethyl α-(1-methylcyclopentyloxyimino)benzeneacetate (0.47g) as a mixture of Z- and E-isomers. Treatment of the mixture with 1M sodium hydroxide solution (1.64ml) in similar manner to that described for Example 3(c) gave the title compound (0.33g), (Found: $M^+$, 275.1503. $C_{16}H_{21}NO_3$ requires M, 275.1521.); $\nu_{max}$ (film) 1735, 1450, 1370, 1330, 1215, 960$cm^{-1}$; $\delta_H$ ($CDCl_3$) 1.1-2.5 (14H,m, incorporating triplet 1.34,J7Hz and singlet 1.46), 1.46 (3H,s), 4.37 (2H,q,J 7Hz), 7.2-8.1 (5H,m); m/z (EI) $M^+$, 275.

(b) α-(Z-1-Methylcyclopentyloxyimino)benzeneacetic acid.-

1M sodium hydroxide solution (2.33ml) was added to ethyl α-(Z-1-methylcyclopentyloxyimino)-benzeneacetate (0.32g) in ethanol (3ml) and the mixture stirred at room temperature for 20h. Extra 1M sodium hydroxide (0.5ml) was added and stirring continued for 96h. The mixture was partitioned between ethyl acetate (20ml) and water (20ml) and 1M hydrochloric acid was added (3ml). The organic phase was washed successively with water (10ml) and saturated sodium chloride solution, dried over magnesium sulphate and concentrated in vacuo. Recrystallization from hexane gave the title compound (0.147g, 51%), m.p. 99-100 °C; (Found: $M^+$, 247.1202. $C_{14}H_{17}NO_3$ requires M, 247.1208.) $\nu_{max}$ (KBr) 1710, 1603, 1498, 1446, 1419, 1254, 960cm$^{-1}$; $\delta_H$ ($CDCl_3$) 1.2-2.5 (11H,m, incoroporating singlet 1.51), 7.2-8.1 (5H,m); m/z (EI) $M^+$, 247.

(c) Sodium 6β-[Z-2-(1-Methylcyclopentyloxyimino)-2-phenylacetamido]penicillanate.-

The title compound was prepared in a manner similar to that described for Example 1(f). Reaction between α-(Z-1-methylcyclopentyloxyimino)benzeneacetic acid (0.212g), oxalyl chloride (114µl), N,N-dimethylformamide (100µl), triethylammonium 6-aminopenicillanate (0.54g) and triethylamine (240µl) followed by HP20SS chromatography [eluting with acetone-water (0:1)-(3:7)] gave the title compound contaminated with the triethylammonium salt. This material was partitioned between dichloromethane and water, and the mixture acidified with 1M hydrochloric acid. The organic phase was separated, the aqueous phase was extracted with dichloromethane and the combined organic phases were then washed with water and with saturated sodium chloride solution, dried over magnesium sulphate and concentrated in vacuo. The acid was dissolved in dilute aqueous sodium hydrogen carbonate solution and chromatographed on HP20SS as before [elution with acetone-water (0:1)-(1:3)]. The title compound was obtained as a lyophilized solid (0.188g, 47%), $\nu_{max}$ (KBr) 1772, 1669, 1604, 1510, 1400, 1319, 953cm$^{-1}$; $\delta_H$ [250MHz, $(CD_3)_2SO$] 1.3-1.85 (15H,m, incorporating singlets 1.42, 1.46 and 1.54), 1.85-2.15 (2H,m), 3.85 (1H,s,3-H), 5.44 (1H,d, J 4Hz,5-H), 5.51 (1H.dd, J 4 and 7.5Hz,6-H), 7.35-7.70 (5H,m), 9.33 (1H,d, J 7.5Hz, $D_2O$ exch., CONH); m/z (FAB) $MH^+$, 468 and $MNa^+$, 490.

## Example 5

### Sodium 6β-[Z-2-(1-Methylcyclopentyloxyimino)-2-(4-trifluoromethylphenyl)acetamido]penicillanate.-

(a) Methyl α-oxo-4-(trifluoromethyl)benzeneacetate.-

The title compound was prepared in a manner similar to that described for Example 1(c). Reaction between chlorotrimethylsilane (3.85ml) and α-oxo-4- (trifluoromethyl)benzeneacetic acid (3.00g) in dry methanol (20ml) followed by 'flash chromatography' [Kieselgel 60, hexane-ethyl acetate (9:1)] gave the title compound (2.94g, 92%), (Found: $M^+$ 232.0347. $C_{10}H_7O_3F_3$ requires M 232.0347.) $\nu_{max}$ (film) 1740, 1695, 1585, 1510, 1415, 1070cm$^{-1}$; $\delta_H$ ($CDCl_3$) 3.96 (3H,s), 7.76 and 8.14 (4H, AA′BB′q, J 8Hz); m/z (EI) $M^+$, 232.

(b) Methyl α-(Z-1-Methylcyclopentyloxyimino)-4-(trifluoromethyl)benzeneacetate.-

The title compound was prepared in a manner similar to that described for Example 1(d). Reaction of methyl α-oxo-4-(trifluoromethyl)benzeneacetate (0.77g) with 1-methylcyclopentyloxyamine hydrochloride (0.50g) followed by chromatography [Kieselgel 60; elution with hexane-ethyl acetate (19:1)] gave the title compound as an oil (0.50g, 46%), (Found: $M^+$ 329.1244. $C_{16}H_{18}NO_3F_3$ requires M 329.1239). $\nu_{max}$ (film) 1745, 1620, 1600, 1435, 1410, 1330, 1070cm$^{-1}$; $\delta_H$ ($CDCl_3$) 1.3-2.3 (11H,m, incorporating singlet 1.47), 3.88 (3H,s), 7.4-7.9 (4H,m); m/z (EI) $M^+$, 329.

(c) α-(Z-1-Methylcyclopentyloxyimino)-4-(trifluoromethyl)benzeneacetic acid.-

The title compound was prepared in a manner similar to that described for Example 1(e). Treatment of methyl α-(Z-1-methylcyclopentyloxyimino)-4(trifluoromethyl)benzeneacetate (0.50g) with 1M sodium hydroxide solution (3.04ml) gave the title compound after recrystallization from hexane (0.13g), m.p. 90-93°C (Found: $M^+$ 315.1094. $C_{15}H_{16}NO_3F_3$ requires M 315.1082). $\nu_{max}$ (KBr) 1718, 1617, 1598, 1410, 1256, 1131, 969, 846cm$^{-1}$; $\delta_H$ (CDCl$_3$) 1.3-2.5 (11H,m, incorporating singlet 1.53), 7.5-7.95 (4H,m), 8.0-8.4 (1H,br); m/z - (EI) $M^+$, 315.

(d) Sodium 6β-[Z-2-(1-Methylcyclopentyloxyimino)-2-(4-trifluoromethylphenyl)acetamido]penicillanate.-

The title compound was prepared in a manner similar to that described for Example 1(f). Reaction between α-(Z-1-methylcyclopentyloxyimino)-4-(trifluoromethyl)benzeneacetic acid (0.384g), oxalyl chloride (0.16ml), N,N-dimethylformamide (0.14ml), triethylammonium 6-aminopenicillanate (0.77g) and triethylamine (0.34ml) followed by chromatography [HP20SS, acetone-water (0:1)-(3:7)] and lyophilization gave the title compound (0.21g). $\nu_{max}$ (KBr) 1772, 1669, 1608, 1509, 1407, 1326, 1168, 958, 847cm$^{-1}$; $\delta_H$ [250MHz, (CD$_3$)$_2$SO] 1.2-1.85 (15H,m, incorporating singlets 1.44, 1.47 and 1.55), 1.85-2.2 (2H,m), 3.90 (1H,s,3-H), 5.46 (1H,d, J 4Hz,5-H), 5.53 (1H, dd, J 4 and 7.5Hz,6-H), 7.82 and 7.76 (4H, AA'BB'q, J 9Hz), 9.44 (1H,d, J 7.5Hz, $D_2O$ exch., CONH); m/z (FAB), $MH^+$, 536.

## Example 6

### Sodium 6β-[Z-2-(2,4-Difluorophenyl)-2-(1-methylcyclohexyloxyimino)acetamido]penicillanate.-

(a) Methyl 2,4-Difluoro-α-oxo-benzeneacetate.-

The title compound was prepared in a manner similar to that described for Example 1(c). Reaction between chlorotrimethylsilane (7.43ml) and 2,4-difluoro-α-oxo-benzeneacetic acid (4.94g) in dry methanol (50ml) followed by 'flash chromatography' [Kieselgel 60, hexane-ethyl acetate (9:1)] gave the title compound (4.89g, 92%) as an almost colourless oil, (Found: $M^+$ 200.0296. $C_9H_6O_3F_2$ requires M 200.0285). $\nu_{max}$ (film) 1755, 1690, 1610, 1595, 1500, 1435, 1320, 1105, 1010cm$^{-1}$; $\delta_H$(CDCl$_3$) 3.93 (3H, s), 6.7-7.2 (2H, m), 7.8-8.2 (1H, m); m/z (EI) $M^+$, 200.

(b) Methyl 2,4-Difluoro-α-(Z-1-methylcyclohexyloxyimino)benzeneacetate.-

The title compound was prepared in a manner similar to that described for Example 1(d). Reaction of methyl 2,4-difluoro-α-oxo-benzeneacetate (0.91g), with 1-methylcyclohexyloxyamine hydrochloride (0.75g) followed by chromatography [Kieselgel 60; hexane-ethyl acetate (19:1)] gave the title compound (0.39g, 28%) as an oil, (Found: $M^+$ 311.1337. $C_{16}H_{19}NO_3F_2$ requires M 311.1333). $\nu_{max}$ (film) 1750, 1615, 1505, 1450, 1435, 1345, 1220, 1105, 980cm$^{-1}$; $\delta_H$(CDCl$_3$) 1.0-2.2 (13H, m, incorporating singlet 1.32), 3.86 (3H, s), 6.6-7.1 (2H, m), 7.55-7.9 (1H, m); m/z (EI) $M^+$, 311.

(c) 2,4-Difluoro-α-(Z-1-methylcyclohexyloxyimino) benzeneacetic acid.-

The title compound was prepared in a manner similar to that described for Example 1(e). Treatment of methyl 2,4-difluoro-α-(Z-1-methylcyclohexyloxyimino)benzeneacetate (0.36g) with 1M sodium hydroxide solution (2.32ml) gave a crude product which was slurried in hexane and filtered to give the title compound (0.17g, 49%), (Found: $M^+$ 297.1182. $C_{15}H_{17}NO_3F_2$ requires M 297.1177). $\nu_{max}$ (KBr) 1719, 1612, 1602, 1502, 1421, 1254, 1103, 978cm$^{-1}$; $\delta_H$(CDCl$_3$) 1.0-2.5 (13H, m, incorporating singlet 1.37), 6.6-7.15 (2H, m), 7.45-7.85 (1H, m), 10.96 (1H, s); m/z (EI) $M^+$, 297.

(d) Sodium 6β-[Z-2-(2,4-Difluorophenyl)-2-(1-methylcyclohexyloxyimino)acetamido]penicillanate

The title compound was prepared in a manner similar to that described for Example 1(f). Reaction between 2,4-difluoro-α-(Z-1-methylcyclohexyloxyimino)benzeneacetic acid (0.30g), oxalyl chloride (133μl), N,N-dimethylformamide (117μl), triethylammonium 6-aminopenicillanate (0.639g) and triethylamine (281μl), then HP20SS chromatography, eluting with acetone-water mixtures (0:1)-(3:7), provided the title compound as a lyophilized solid (0.31g, 59%), $\nu_{max}$ (KBr) 1773, 1671, 1610, 1507, 1401, 1142, 1100, 974, 849cm$^{-1}$; $\delta_H$-[250MHz, $(CD_3)_2SO$] 1.0-1.7 (17H, m, incorporating singlets at 1.27, 1.47 and 1.55), 1.7-2.0 (2H, m), 3.87 (1H, s, 3-H), 5.43 (1H, d, J 4Hz, 5-H), 5.55 (1H, dd, J 4Hz and 8.3Hz, 6-H), 7.1-7.27 (1H, m), 7.27-7.43 (1H, m), 7.57-7.74 (1H, m), 9.16 (1H, d, J 8.3Hz, $D_2O$ exch., CONH); m/z (FAB) $MH^+$, 518 and $MNa^+$, 540.

Example 7

Sodium 6β-[Z-2-(1-Ethylcyclopentyloxyimino)-2-(2-fluorophenyl)acetamido]penicillanate

(a) Ethyl 2-Fluoro-α-oxo-benzeneacetate

1.6M n-Butyllithium solution in hexane (8.59ml) was added over 10 min. to a solution of 2-fluorobromobenzene (1.43ml) in sodium dried diethyl ether (20ml) cooled to -69°C under an argon atmosphere. During the addition the temperature was kept below -60°C. This mixture was added to a solution of ethyl α-oxo-1H- imidazole-1-acetate (11.52g) in sodium dried diethyl ether (110ml) cooled to -47°C under an argon atmosphere over 3 min via a double tipped needle. The mixture was stirred at -40°C for 15 min, then allowed to warm up. The mixture was mixed with water (150ml) and the phases separated. The aqueous phase was extracted with diethyl ether (100ml). The combined organic phases were washed with water (2x50ml) and with saturated sodium chloride solution (50ml), dried over magnesium sulphate and concentrated in vacuo. The crude product was chromatographed [Kieselgel 60, hexane-ethyl acetate (19:1)-(9:1)] to give the title compound (1.41g, 55%) as an oil. (Found: $M^+$ 196.0537. $C_{10}H_9O_3F$ requires M 196.0536). $\nu_{max}$ (film) 1745, 1695, 1615, 1585, 1485, 1460, 1200, 1020cm$^{-1}$; $\delta_H(CDCl_3)$ 1.38 (3H, t, J 7Hz) 4.40 (2H, q, J 7Hz), 7.0-7.45 (2H, m), 7.45-7.8 (1H, m), 7.8-8.1 (1H, m); m/z CI ($NH_3$ gas) $MNH_4^+$, 214.

(b) Ethyl 2-Fluoro-α-(Z-hydroxyimino)benzeneacetate

Hydroxylamine hydrochloride (0.89g) was added to ethyl 2-fluoro-α-oxo-benzeneacetate (2.46g) in ethanol (10ml), followed by water (1ml). The reaction mixture was stirred for 18hrs, then partitioned between ethyl acetate (25ml) and water (15ml). The aqueous phase was extracted with ethyl acetate (15ml) and the combined organic extracts washed with water and saturated sodium chloride solution, dried over magnesium sulphate and concentrated in vacuo. The crude product (3.0g) was chromatographed [Kieselgel 60, ethyl acetate-hexane (1:9)-(1:4)] to give the E isomer (0.98g) and the title compound (1.51g, 57%) (Found: $M^+$ 211.0651. $C_{10}H_{10}NO_3F$ requires M 211.0645); $\nu_{max}$ (film) 3375(br), 1730, 1615, 1595, 1490, 1265 and 955cm$^{-1}$; $\delta_H(CDCl_3)$ 1.33 (3H, t, J 7Hz), 4.38 (2H, q, J 7Hz), 6.8-7.75 (4H, m), 10.09 (1H, s); m/z (EI) $M^+$, 211.

(c) Ethyl 2-Fluoro-α-(Z-1-ethylcyclopentyloxyimino)benzeneacetate

Boron trifluoride etherate (0.29ml) was added to ethyl 2-fluoro-α-(Z-hydroxyimino)benzeneacetate (0.509g), 1-ethylcyclopentanol (0.27g) and molecular sieves (3.5g) in dry dichloromethane (15ml). The mixture was heated to reflux and heating and stirring continued overnight. The apparatus was previously flushed with argon, and the reaction was carried out under a silica gel drying tube. The reaction mixture was allowed to cool, then filtered through celite. The filtrate was washed successively with 1M sodium hydrogen carbonate solution (20ml), water (20ml), saturated sodium chloride solution (20ml), dried over magnesium sulphate and concentrated in vacuo. The crude product was chromatographed [Kieselgel 60, hexane-ethyl acetate (9:1-3:1)] to give the title compound (0.42g, 57%), (Found: $M^+$, 307.1573. $C_{17}H_{22}NO_3F$ requires M 307.1584); $\nu_{max}$ (film) 1740, 1615, 1600, 1490, 1455, 1370, 1265 and 965cm$^{-1}$; $\delta_H(CDCl_3)$ 0.91 (3H, t, J 8Hz), 1.2-2.5 (13H, m, incorporating triplet 1.33 J 6Hz), 4.35 (2H, q, J 6Hz), 6.90-7.6 (3H, m), 7.6-7.9 (1H, m); m/z (EI) $M^+$, 307.

(d) 2-Fluoro-α-(Z-1-ethylcyclopentyloxyimino)benzeneacetic acid

The title compound was prepared in a manner similar to that described for Example 1(e). Treatment of ethyl 2-fluoro-α-(Z-1-ethylcyclopentyloxyimino)benzeneacetate (0.36g) in ethanol (10ml) with 1M sodium hydroxide solution (2.35ml) gave the title compound after recrystallization from hexane (0.20g, 61%), m.p. 73-74° C (Found: $M^+$ 279.1280. $C_{15}H_{18}NO_3F$ requires 279.1271). $\nu_{max}$ (KBr) 1718, 1613, 1596, 1487, 1455, 1265, 967 and 765cm$^{-1}$; $\delta_H(CDCl_3)$ 0.94 (3H, t, J 7.5Hz), 1.45-1.95 (8H, m, incorporating quartet, 1.87 J 7.5Hz), 1.95-2.2 (2H, m), 7.04-7.25 (2H, m), 7.34-7.5 (1H, m), 7.53-7.7 (1H, m); m/z (EI) $M^+$, 279.

(e) Sodium 6β-[Z-2-(1-Ethylcyclopentyloxyimino)-2-(2-fluorophenyl)acetamido]penicillanate

The title compound was prepared in a manner similar to that described for Example 1(f). Reaction between 2-fluoro-α-(Z-1-ethylcyclopentyloxyimino)benzeneacetic acid (0.177g), oxalyl chloride (0.084ml), N,N-dimethylformamide (0.074g), triethylammonium 6-aminopenicillanate (0.40g) and triethylamine (0.35ml), followed by chromatography[HP20SS, tetrahydrofuran-water (0:1)-(2:9)] and lyophilization gave the title compound (0.16g, 50%), $\nu_{max}$ (KBr) 1773, 1671, 1611, 1512, 1401, 1319, 949, 759cm$^{-1}$; $\delta_H$ [250MHz, $(CD_3)_2SO$] 0.86 (3H, t, J 7.5Hz) 1.36-1.86 (14H, m, incorporating singlets 1.46 and $\delta_H$[250MHz, $(CD_3)_2SO$] 0.86 (3H, t, J 7.5Hz), 1.36-1.86 (14H, m, incorporating singlets 1.46 and 1.54), 1.86-2.10 (2H, m), 3.85 (1H, m), 5.43 (1H, d, J 4Hz), 5.51 (1H, dd, J 4Hz and 8Hz), 7.17-7.37 (2H, m), 7.37-7.65 (2H, m), 9.05 (1H, d, J 8Hz, $D_2O$ exch., CONH); m/z (FAB) $MH^+$, 500.

Example 8

Sodium 6β-[Z-2-(4-Chlorophenyl)-2-(1-methylcyclopentyloxyimino)acetamido]penicillanate

(a) Methyl 4-Chloro-α-oxo-benzeneacetate

A solution of 4-chlorophenyl magnesium bromide in tetrahydrofuran (50ml) prepared from 4-bromochlorobenzene (4.83g) was added dropwise over 1hr to a solution of methyl α-oxo-1H-imidazole-1-acetate (3.85g) in freshly distilled tetrahydrofuran (75ml) under argon. The temperature was maintained between -60° C and -40° C. The reaction mixture was allowed to warm up over 2hr, then poured into an ice/water mixture ( 200g) and extracted with diethyl ether (2x100ml). The combined organic extracts were washed with saturated sodium chloride solution (50ml), dried over magnesium sulphate and concentrated in vacuo. The crude material was flash chromatographed [Kieselgel 60, ethyl acetatehexane (1:9)] to give the title compound (2.14g, 43%), m.p. 55-59° C (Found 198.0082. $C_9H_7O_3Cl$ requires 198.0084). $\nu_{max}$ (KBr) 1727, 1685, 1588, 1329, 1213 and 1005cm$^{-1}$; $\delta_H(CDCl_3)$ 3.94 (3H, s), 7.45 and 7.95 (4H, AA′BB′q, J 8Hz); m/z (EI) $M^+$, 198.

(b) Methyl 4-Chloro-α-(Z-1-methylcyclopentyloxyimino)benzeneacetate

The title compound was prepared in a manner similar to that described for Example 1(d). Reaction of methyl 4-chloro-α-oxo-benzeneacetate (0.43g) with 1-methylcyclopentyloxyamine hydrochloride (0.33g), followed by chromatography [Kieselgel 60; elution with hexane-ethyl acetate (19:1)] gave the title compound (0.28g, 44%) as an oil, (Found: 295.0975. $C_{15}H_{18}NO_3Cl$ requires 295.0975). $\nu_{max}$ (film) 1745, 1605, 1495, 1335, 1225, 965cm$^{-1}$; $\delta_H(CDCl_3)$ 1.2-2.3 (m, incorporating singlet, 1.45), 3.87 (3H, s), 7.31 and 7.48 (4H, AA′BB′q, J 9Hz); m/z (EI) $M^+$, 295.

(c) 4-chloro-α-(Z-1-methylcyclopentyloxyimino)benzeneacetic acid

The title compound was prepared in a manner similar to that described for Example 1(e). Treatment of methyl 4-chloro-α-(Z-1-methylcyclopentyloxyimino)benzeneacetate (0.28g) in methanol (3ml) with 1M sodium hydroxide solution (2.88ml) gave the title compound (0.14g, 52%), m.p. 109-111.5° C (Found: C,

59.87; H, 5.85; N, 5.09; Cl, 12.61%; M$^+$, 281.0828. $C_{14}H_{16}NO_3Cl$ requires C, 59.82; H, 5.74; N, 4.98; Cl, 12.61%; M, 281.0819). $\nu_{max}$ (KBr) 1717, 1603, 1494, 1400, 1250, 1093, 961, 833cm$^{-1}$; $\delta_H$[250MHz, $CDCl_3$] 1.45-1.95 (9H, m, incorporating singlet 1.52), 1.95-2.25 (2H, m), 7.38 and 7.60 (4H, AA′BB′q, J 8.6Hz); m/z - (EI) M$^+$, 281.

(d) Sodium 6β-[Z-2-(4-Chlorophenyl)-2-(1-methylcyclopentyloxyimino)acetamido]penicillanate

The title compound was prepared in a manner similar to that described for Example 1(f). Reaction between 4-choro-α-(Z-1-methylcyclopentyloxyimino)benzeneacetic acid (0.142g), oxalyl chloride (67μl), N,N-dimethylformamide (59μl) triethylammonium 6-aminopenicillanate (0.32g), and triethylamine (140μl), followed by chromatography [HP20SS, acetone-water (0:1)-(3:7)] and lyophilization gave the title compound (0.171g, 68%), $\nu_{max}$ (KBr) 1773, 1671, 1617, 1517, 1401, 1092, 950cm$^{-1}$; $\delta_H$ [250MHz, $(CD_3)_2SO$] 1.3-1.85 (m, incorporating singlets 1.42, 1.46 and 1.53), 1.85-2.1 (2H, m), 3.83 (1H, s, 3-H), 5.43 (1H, d, J 4Hz), 5.49 (1H, dd, J 4Hz and 7.5Hz), 7.51 and 7.57 (4H, AA′BB′q, J 9Hz), 9.36 (1H, d, J 7.5Hz, $D_2O$ exch., CONH); m/z (FAB) MNa$^+$, 524 and MH$^+$, 502.

## Example 9

### Sodium 6β-[Z-2-(4-Fluorophenyl)-2-(1-methylcyclopentyloxyimino)acetamido]penicillanate

(a) Methyl 4-Fluoro-α-(Z-1-methylcyclopentyloxyimino)benzeneacetate

Chlorotrimethylsilane (1.95ml) was added to 4-fluoro-α-oxo-benzeneacetic acid (1.17g) in dry methanol and allowed to react at room temperature under an argon atmosphere for 7 days. The reaction mixture was concentrated in vacuo. Flash chromatography [Kieselgel 60, hexane-ethyl acetate (9:1)] gave a semi-solid (0.61g). This material (0.33g) in methanol (3ml) was treated with 1-methylcyclopentyloxyamine hydrochloride

(0.27g) and the reaction stirred at room temperature for 24hr. The mixture was partitioned between ethyl acetate (20ml) and water (10ml) and the aqueous phase extracted with ethyl acetate (10ml). The combined organic phases were washed with water and with saturated sodium chloride solution, dried over magnesium sulphate and concentrated in vacuo. Chromatography [Kieselgel 60, hexane-ethyl acetate (19:1)] gave the title compound (0.16g, 32%). $\nu_{max}$ (film) 1745, 1614, 1515, 1440, 1335, 965cm$^{-1}$;$\delta_H$($CDCl_3$) 1.1-2.5 (11H, m, incorporating singlet 1.44), 3.87 (3H, s), 6.9-7.3 (2H, m), 7.4-7.7 (2H, m).

(b) 4-Fluoro-α-(Z-1-methylcyclopentyloxyimino)benzeneacetic acid

The title compound was prepared in a manner similar to that descibed for Example 1(e). Treatment of methyl 4-fluoro-α-(Z-1-methylcyclopentyloxyimino)benzeneacetate (0.15g) in methanol (3ml) with 1M sodium hydroxide solution (1.10ml) gave the title compound (0.10g, 70%), m.p. 97.5-102° C. (Found: C, 63.20; H, 6.06; N, 5.26%; M$^+$, 265.1123. $C_{14}H_{16}NO_3F$ requires C, 63.43; H, 6.08; N, 5.28%; M, 265.1114). $\nu_{max}$ - (KBr) 1718, 1609, 1600, 1510, 1405, 952, 843cm$^{-1}$; $\delta_H$($CDCl_3$) 1.1-2.5 (11H, m, incorporating singlet 1.50), 6.8-8.0 (4H, m), 10.1-10.35 (1H, s); m/z (EI) M$^+$, 265.

(c) Sodium 6β-[Z-2-(4-Fluorophenyl)-2-(1-methylcyclopentyloxyimino)acetamido]penicillanate

The title compound was prepared in a manner similar to that described for Example 1(f). Reaction between 4-fluoro-α-(Z-1-methylcyclopentyloxyimino)benzeneacetic acid (0.077g), oxalyl chloride (38μl), N-N-dimethylformamide (34μl), triethylammonium 6-aminopenicillanate (0.18g) and triethylamine (81μl) followed by chromatography [HP20SS, acetone-water (0:1)-(3:7), twice] and lyophilization gave the title compound (0.07g, 50%), $\nu_{max}$ (KBr) 1773, 1670, 1604, 1508, 1232, 954, 840cm$^{-1}$; $\delta_H$[250MHz, $(CD_3)_2SO$] 1.3-1.85 (m, incorporating singlets 1.41, 1.45 and 1.53), 1.9-2.1 (2H, m), 3.82 (1H, s, 3-H), 5.42 (IH, d, J 4Hz), 5.48 (1H, dd, J 4Hz and 7.5Hz), 7.2-7.4 (2H, m), 7.55-7.7 (2H, m), 9.33 (1H, d, J 7.5Hz, $D_2O$ exch.,

CONH); m/z (FAB) $MNa^+$, 508 and $MH^+$ 486.

Example 10

Sodium 6β-[Z-2-(2,6-Difluorophenyl)-2-(1-methylcyclopentyloxyimino)acetamido]penicillanate

(a) Methyl 2,6-Difluoro-α-oxo-benzeneacetate

The title compound was prepared in a manner similar to that described for Example 1(c). Reaction between chlorotrimethylsilane (3.77ml) and 2,6-difluoro-α-oxo-benzeneacetic acid (2.50g) in dry methanol (15ml) followed by chromatography [Kieselgel 60, hexane-ethyl acetate (4:1)] gave the title compound - (2.53g, 94%), (Found: $M^+$ 200.0281. $C_9H_6O_3F_2$ requires M 200.0285). $\nu_{max}$ (film) 1750, 1715, 1625, 1595, 1470, 1005cm$^{-1}$; $\delta_H$($CDCl_2$) 3.94 (3H, s), 6.8-7.3 (2H, m), 7.35-7.85 (1H, m); m/z CI, ($NH_3$ gas) $MNH_4^+$, 218.

(b) Methyl 2,6-Difluoro-α-(Z-hydroxyimino)benzeneacetate

The title compound was prepared in a manner similar to that described for Example 7(b). Treatment of methyl 2,6-difluoro-α-oxo-benzeneacetate (0.40g) in methanol (2ml) and water (0.2ml) with hydroxylamine hydrochloride (0.142g) followed by chromatography [Kieselgel 60, ethyl acetate-hexane (1:9)-(1:0)] gave the E-isomer (0.138g) and the title compound (0.186g, 43%). (Found: $M^+$ 215.0392. $C_9H_7NO_3F_2$ requires M 215.0394). $\nu_{max}$ (KBr) 1753, 1624, 1570, 1469, 1433, 1217, 999, 796cm$^{-1}$; $\delta_H$($CDCl_3$) 3.85 (3H, s), 6.75-7.2 (2H, m), 7.2-7.65 (1H, m), 12.23 (1H, s); m/z (EI) $M^+$, 215.

(c) Methyl 2,6-Difluoro-α-(Z-1-methylcyclopentyloxyimino)benzeneacetate

The title compound was prepared in a manner similar to that described for Example 7(c). Reaction of boron trifluoride etherate (0.095ml), 1-methylcyclopentanol (0.098g), and methyl 2,6-difluoro-α-(Z-hydroxyimino)benzeneacetate (0.168g) in dichloromethane (4ml) in the presence of 3A molecular sieves (1.1g) followed by chromatography [Kieselgel 60, hexane-ethyl acetate (9:1)] gave the title compound (0.146g, 63%) as a colourless oil. (Found: $MH^+$, 298.1255. $C_{15}H_{18}NO_3F_2$ requires MH, 298.1255). $\nu_{max}$ (film) 1740, 1630, 1595, 1575, 1470, 1375, 795cm$^{-1}$; $\delta_H$($CDCl_3$) 1.4-1.9 (m, incorporating singlet 1.47), 1.97-2.15 (2H, m), 3.85 (3H, s), 6.88-7.02 (2H, m), 7.24-7.42 (1H, m); m/z CI ($NH_3$ gas) $MNH_4^+$, 315 and $MH^+$, 298.

(d) 2,6-Difluoro-α-(Z-1-methylcyclopentyloxyimino)benzeneacetic acid

The title compound was prepared in a manner similar to that described for Example 1(e). Treatment of methyl 2,6-difluoro-α-(Z-1-methylcyclopentyloxyimino)benzeneacetate (0.17g) with 1M sodium hydroxide solution (1.16ml) gave the title compound (0.07g, 43%) after recrystallization from hexane, m.p. 62-64°C (Found: $M^+$ 283.1020. $C_{14}H_{15}NO_3F_2$ requires M, 283.1020). $\nu_{max}$ (KBr) 1714, 1655, 1628, 1466, 1382, 1239, 1002, 960, 782cm$^{-1}$; $\delta_H$(250MHz, $CDCl_3$) 1.55 (3H, s), 1.6-1.9 (6H, m), 2.05-2.28 (2H, m), 6.9-7.05 (2H, m), 7.3-7.5 (1H, m), 10.5-11.25(br); m/z (EI) $M^+$ 283.

(e) Sodium 6β-[Z-2-(2,6-Difluorophenyl)-2-(1-methylcyclopentyloxyimino)acetamido]penicillanate

The title compound was prepared in a manner similar to that described for Example 1(f). Reaction between 2,6-difluoro-α-(Z-1-methylcyclopentyloxyimino)benzeneacetic acid (0.116g), oxalyl chloride (0.054ml), N,N-dimethylformamide (0.048g), triethylammonium 6-aminopenicillanate (0.260g) and triethylamine (0.228ml) gave the penicillin acid which was taken up in 1,4-dioxan (10ml) and water (10ml). The pH of the chilled mixture was adjusted to 6 with dilute sodium hydroxide solution and the crude penicillin sodium salt (0.20g) obtained by lyophilization. The title compound (0.121g, 59%) was obtained after chromatography [HP20SS, tetrahydrofuran-water (0:1)-(1:4)] and lyophilization. $\nu_{max}$ (KBr) 1774, 1670,

1626, 1516, 1466, 1401, 1237, 1002, 951, 791cm$^{-1}$; $\delta_H$[250MHz, $(CD_3)_2SO$] 1.35-1.85 (15H, m, incorporating singlets at 1.43, 1.49 and 1.55), 1.95-2.20 (2H, m), 3.92 (1H, s), 5.51 (1H. d, J 4Hz. 5-H). 5.58 (1H, dd, J 4Hz and 8.5Hz, 6-H), 7.1-7.3 (2H, m), 7.46-7.64 (1H. m), 8.73 (1H, d, J 8.5Hz, $D_2O$ exch., CONH); m/z - (FAB), $MH^+$ 504.

Example 11

Sodium 6β-[Z-2-(3-Fluorophenyl)-2-(1-methylcyclopentyloxyimino)acetamido]penicillanate.

(a) Ethyl 3-Fluoro-α-oxo-benzeneacetate.

The title compound was prepared in a manner similar to that described for Example 8(a). Reaction of 3-fluorophenyl magnesium bromide prepared from 3-bromofluorobenzene (2.19g) with ethyl α-oxo-1H-imidazole-1-acetate (4.18g) followed by chromatography[twice, Kieselgel 60, hexane-ethyl acetate (19:1)] gave the title compound (1.35g, 55%) as an oil, (Found: $M^+$ 196.0538. $C_{10}H_9O_3F$ requires M 196.0536). $\nu_{max}$(film) 1740, 1695, 1595, 1315, 1240, 1150, 1030cm$^{-1}$; $\delta_H$($CDCl_3$) 1.41 (3H, t, J 7Hz), 4.44 (2H, q, J 7Hz), 7.2-8.1 (4H, m); m/z (EI) $M^+$ 196.

(b) Ethyl 3-Fluoro-α-(Z-hydroxyimino)benzeneacetate.

The title compound was prepared in a manner similar to that described for Example 7(b). Treatment of ethyl 3-fluoro-α-oxo-benzeneacetate (0.48g) in ethanol (3ml) and water (0.3ml) with hydroxylamine hydrochloride (0.17g) followed by chromatography [Kieselgel 60, hexane-ethyl acetate (19:1)-(0:1)] gave the E isomer (0.15g) and the title compound (0.31g, 60%). (Found: $M^+$ 211.0647. $C_{10}H_{10}NO_3F$ requires M 211.0645). $\nu_{max}$(film) 3400(br), 1730, 1590, 1450, 1245, 1180, 1045, 970cm$^{-1}$; $\delta_H$($CDCl_3$) 1.38 (3H, t, J 7Hz), 4.44 (2H, g, J 7Hz), 6.9-7.8 (4H, m), 9.16 (1H, br,s); m/z (EI) $M^+$, 211.

(c) Ethyl 3-Fluoro-α-(Z-1-methylcyclopentyloxyimino)benzeneacetate.

The title compound was prepared in a manner similar to that described for Example 7(c). Reaction of boron trifluoride etherate (0.38ml), 1-methylcyclopentanol (0.31g) and ethyl 3-fluoro-α-(Z-hydroxyimino)-benzeneacetate (0.65g) in dichloromethane (12ml) in the presence of 3A molecular sieves (5g) followed by chromatography [Kieselgel 60, hexane-ethyl acetate (19:1)] gave the title compound (0.63g, 70%) as an oil. (Found: $M^+$ 293.1425. $C_{16}H_{20}NO_3F$ requires M 293.1427). $\nu_{max}$(film) 2970, 1740, 1580, 1445, 1235, 970, 885cm$^{-1}$; $\delta_H$($CDCl_3$) 1.2-2.2 (m, incorporating triplet 1.36, J 7Hz), 4.39 (2H, q, J 7Hz), 6.9-7.8 (4H, m); m/z - (EI) $M^+$, 293.

(d) 3-Fluoro-α-(Z-1-methylcyclopentyloxyimino)benzeneacetic acid.

1M sodium hydroxide solution (3.84ml) was added to ethyl 3-fluoro-α-(Z-1-methylcyclopentyloxyimino)-benzeneacetate (0.56g) in ethanol (10ml) and stirred at room temperature overnight. Additional 1M sodium hydroxide solution (0.38ml) was added and after a further 4.5h the mixture was partitioned between dichloromethane and water. The pH of the aqueous phase was adjusted to ~6 with 1M hydrochloric acid, and then to 2.25 in the presence of ethyl acetate (20ml). The aqueous phase was extracted with ethyl acetate (20ml) and the combined organic extracts washed succesively with water (20ml) and with saturated brine (20ml), dried over magnesium sulphate and concentrated in vacuo. The title compound was obtained after recrystallization from hexane (0.37g, 73%), (Found: $M^+$ 265.1116. $C_{14}H_{16}NO_3F$ requires 265.1114). $\nu_{max}$(KBr) 1713, 1602, 1578, 1489, 1433, 1420, 1261, 1186, 982cm$^{-1}$; $\delta_H$(250MHz, $CDCl_3$) 1.3-1.9 (9H, m, incorporating singlet 1.52), 1.9-2.2 (2H, m) 7.0-7.2 (1H, m), 7.2-7.5 (3H, m); m/z (EI) $M^+$, 265.

(e) Sodium 6β-[Z-2-(3-Fluorophenyl)-2-(1-methylcyclopentyloxyimino)acetamido]penicillanate.

The title compound was prepared in a manner similar to that described for Example 1(f). Reaction between 3-fluoro-α-(Z-1-methylcyclopentyloxyimino)benzeneacetic acid (0.35g), oxalyl chloride (0.174ml), N,N-dimethylformamide (0.153ml), triethylammonium 6-aminopenicillanate (0.836g) and triethylamine (0.367ml) gave the penicillin acid. Treatment of the penicillin acid in t-butanol (15ml) and water (15ml) with dilute sodium hydroxide solution, followed by lyophilization gave the title compound (0.54g, 84%), $\nu_{max}$(KBr) 1773, 1675, 1597, 1521, 1399, 1327, 966cm$^{-1}$; $\delta_H$[250MHz, $(CD_3)_2SO$] 1.3-1.85 (15H, m, incorporating singlets 1.42, 1.46 and 1.54), 1.85-2.15 (2H, m), 3.86 (1H, s, 3-H), 5.4-5.55 (2H, m), 7.2-7.6 (4H, m), 9.41 (1H, d, J 7.2Hz, $D_2O$ exch., CONH); m/z (FAB) $MH^+$, 486 and $MNa^+$, 508.

Example 12

Sodium 6β-[Z-2-(2-chloro-6-fluorophenyl)-2-(1-methyl cyclopentyloxyimino)acetamido]penicillanate.

(a) Ethyl 2-Chloro-6-fluoro-α-oxo-benzeneacetate.

1.7M n-Butyllithium hexane solution (4.47ml) was added over 30 min to 3-chlorofluorobenzene (0.79ml) in freshly distilled tetrahydrofuran (20ml) at -60°C under an argon atmosphere. The mixture was stirred at -60°C for 50 min, then added to ethyl α-oxo-1H-imidazole-1-acetate (3.98g) in freshly distilled tetrahydrofuran (40ml) under argon at -65°C via a double tipped needle over 2-3min. After a further 15 min, the cooling was removed and the mixture allowed to warm up over 1 hr. 1M Ammonium chloride solution (20ml), ethyl acetate (20ml) and saturated sodium chloride solution (20ml) were added, and the phases separated. The aqueous phase was extracted with ethyl acetate (20ml), the combined organic extracts washed successively with water (2x20ml) and saturated sodium chloride solution (20ml), dried over magnesium sulphate and concentrated in vacuo. The crude product was chromatographed [Merck Kieselgel 60, 1:1 7729-9385, hexane-ethyl acetate (19:1)] to give the title compound (1.42g, 82%) as an oil. (Found: $M^+$ 230.0146. $C_{10}H_8O_3FCl$ requires M 230.0146). $\nu_{max}$(film) 1735(br), 1600, 1575, 1455, 1370, 1020, 910cm$^{-1}$; $\delta_H$( $CDCl_3$) 1.36 (3H, t, J 7Hz), 4.38 (2H, q, J 7Hz), 6.9-7.8 (3H, m); m/z (EI) $M^+$, 230.

(b) Ethyl 2-Chloro-6-fluoro-α-(Z-hydroxyimino)benzeneacetate.

The title compound was prepared in a manner similar to that described for Example 7(b). Treatment of ethyl 2-chloro-6-fluoro-α-oxo-benzeneacetate (1.00g) in ethanol (5ml) and water (0.5ml) with hydroxylamine hydrochloride (0.30g) followed by chromatography [Kieselgel 60, hexane-ethyl acetate (9:1)-(4:1)] gave the E-isomer (0.702g) and the title compound (0.155g, 15%), m.p. 61-63°C. (Found: $M^+$ 245.0245. $C_{10}H_9NO_3FCl$ requires M 245.0255). $\nu_{max}$(KBr) 3332(br), 1690, 1613, 1570, 1448, 1319, 1233, 956, 793cm$^{-1}$; $\delta_H$($CDCl_3$) 1.28 (3H, t, J 7Hz), 4.34 (2H, q, J 7Hz), 7.02-7.18 (1H, m), 7.22-7.44 (2H, m); m/z (EI) $M^+$, 245.

(c) Ethyl 2-Chloro-6-fluoro-α-(Z-1-methylcyclopentyloxyimino)benzeneacetate.

The title compound was prepared in a manner similar to that described for Example 7(b). Reaction of boron trifluoride etherate (0.068ml), 1-methylcyclopentanol (0.062g) and ethyl 2-chloro-6-fluoro-α-(Z-hydroxyimino)benzeneacetate (0.138g) in dichloromethane (4ml) in the presence of 3A molecular sieves (0.71g) followed by flash chromatography [Kieselgel 60, hexane-ethyl acetate (9:1)] gave the title compound - (0.099g, 54%). $\nu_{max}$(film) 1730, 1610, 1575, 1450, 1025, 960, 900, 785cm$^{-1}$; $\delta_H$($CDCl_3$) 1.1-2.3 (m, incorporating triplet 1.27, J 6.5Hz and singlet 1.45), 4.28 (2H, q, J 6.5Hz), 6.8-7.5 (3H, m); m/z CI (isobutane) $MH^+$, 328.

(d) 2-Chloro-6-fluoro-α-(Z-1-methylcyclopentyloxyimino)benzeneacetic acid.

The title compound was prepared in a manner similar to that described for Example 1(e). Treatment of ethyl 2-chloro-6-fluoro-α-(Z-1-methylcyclopentyloxyimino)benzeneacetate (0.088g) in ethanol (2ml) with 1M

sodium hydroxide solution (0.54ml) gave the title compound (0.070g, 87%) after trituration from hexane. $\nu_{max}$(KBr) 1716, 1697, 1604, 1565, 1446, 1250, 972, 896, 789cm$^{-1}$; $\delta_H$($CDCl_3$) 1.4-1.95 (m,incorporating singlet 1.56), 2.05-2.30 (2H, m), 7.0-7.15 (1H, m), 7.23-7.32 (1H, m), 7.32-7.45 (1H, m), 10.95 (1H, br); m/z CI (isobutane) $MH^+$ 300.

(e) Sodium 6β-[Z-2-(2-Chloro-6-fluorophenyl)-2-(1-methylcyclopentyloxyimino)acetamido]penicillanate.

The title compound was prepared in a manner similar to that described for Example (1f). Reaction between 2-chloro-6-fluoro-α-(Z-1-methylcyclopentyloxyimino)benzeneacetic acid (0.06g), oxalyl chloride (26μl), N,N-dimethylformamide (23μl), triethylammonium 6-aminopenicillanate and triethylamine (70μl) followed by an acidic work-up gave the free acid. The title compound (0.082g, 79%) was obtained following treatment of a t-butanol/water solution of the acid with dilute sodium hydroxide solution, partial concentration and subsequent lyophilization. $\nu_{max}$(KBr) 1775, 1670, 1609, 1516, 1448, 950, 898, 785cm$^{-1}$; $\delta_H$[250MHz, $(CD_3)_2SO$] 1.35-1.8 (m, incorporating singlets 1.44, 1.50, and 1.54). 1.95-2.2 (2H, m). 3.94 (1H, s). 5.54 (1H. d, J 4Hz, 5-H), 5.60 (1H, dd, J 4 and 9Hz, 6-H), 7.23-7.6 (3H, m) 8.58 (1H, d, J 9Hz, $D_2O$ exch., CONH); m/z (FAB), $MH^+$, 520.

Example 13

Sodium 6β-[Z-2-(2-Fluorophenyl)-2-(1-methylcyclooctyl oxyimino)acetamido]penicillanate.

(a) Ethyl 2-Fluoro-α-(Z-1-methylcyclooctyloxyimino)benzeneacetate.

The title compound was prepared in a manner similar to that described for Example 7(c). Reaction of boron trifluoride etherate (0.13ml), 1-methylcyclooctanol (0.15g), and ethyl 2-fluoro-α-(Z-hydroxyimino)-benzeneacetate (0.22g) in dry dichloromethane (4ml) in the presence of molecular sieves (1.5g) followed by chromatography [Kieselgel 60, hexane-ethyl acetate (39:1)] gave the title compound (0.064g, 18%). $\nu_{max}$-(film) 2935, 2865, 1740, 1490, 1455, 1265, 1217, 970, 765cm$^{-1}$; $\delta_H$($CDCl_3$) 1.1-2.3 (m, incorporating triplet 1.34, J 7Hz), 4.35 (2H, q, J 7Hz), 6.8-7.5 (3H, m), 7.6-7.9 (1H, m); m/z CI ($NH_3$ gas) $MH^+$, 336.

(b) 2-Fluoro-α-(Z-1-methylcyclooctyloxyimino)benzeneacetic acid.

The title compound was prepared in a manner similar to that described for Example 3(d). Treatment of ethyl 2-fluoro-α-(Z-1-methylcyclooctyloxyimino)benzeneacetate (0.055g) with 1M sodium hydroxide solution (0.36ml and 0.14ml) in ethanol (2.5ml) gave the title compound as an oil which crystallized on standing in the freezer (0.050g, 99%), m.p. 79-82°C. $\nu_{max}$(KBr) 1718, 1487, 1272, 967, 765cm$^{-1}$; $\delta_H$($CDCl_3$) 1.2-1.95 (m, incorporating singlet 1.41), 2.0-2.25 (2H, m), 7.05-7.25 (2H, m), 7.35-7.55 (2H, m), 10.4(br); m/z CI (isobutane) $MH^+$, 308.

(c) Sodium 6β-[Z-2-(2-Fluorophenyl)-2-(1-methylcyclooctyloxyimino)acetamido]penicillanate.

Triethylamine (78μl) was added to 6-aminopenicillanic acid (0.060g) in dry dichloromethane (0.5ml) and stirred for 1 h. Oxalyl chloride (18μl) was added to N,N-dimethylformamide (16μl) in dry dichloromethane (0.5ml) at -20°C under argon, and stirred for 11 min. 2-Fluoro-α-(Z-1-methylcyclooctyloxyimino)-benzeneacetic acid (0.043g) in dry dichloromethane (1.0ml) was added, and the mixture stirred for about 10 min with cooling. The triethylammonium 6-aminopenicillanate/triethylamine solution was briefly chilled, then added to the above mixture. After a further 9 min, the cooling was removed and the reaction mixture was stirred at room temperature for 0.6 h. The reaction was worked up in a similar manner to that described for Example 1(f) to give the penicillin acid. Treatment of the penicillin acid in t-butanol (5ml) and water (5ml) with dilute sodium hydroxide solution followed by partial concentration and lyophilization gave the title compound (0.05g, 68%). $\nu_{max}$(KBr) 2924, 2856, 1775, 1670, 1611, 1512, 955, 758cm$^{-1}$; $\delta_H$[250MHz,$(CD_3)_2SO$] 1.1-1.85 (21H, m, incorporating singlets 1.29, 1.47 and 1.55), 1.85-2.15 (2H, m), 3.88 (1H, s), 5.44 (1H,

d, J 4Hz), 5.53 (1H, dd, J 4Hz and 8Hz), 7.16-7.38 (2H, m), 7.38-7.67 (2H, m), 9.05 (1H, d, J 8Hz, $D_2O$ exch., CONH); m/z (FAB) $MNa^+$, 550 and $MH^+$, 528.

## Example 14

### Sodium 6β-[Z-2-(2-Fluorophenyl)-2-(1-heptylcyclopentyloxyimino)acetamido]penicillanate.

(a) Ethyl 2-Fluoro-α-(Z-1-heptylcyclopentyloxyimino)benzeneacetate.

The title compound was prepared in a manner similar to that described for Example 7(c). Reaction of boron trifluoride etherate (0.12ml), 1-heptylcyclopentanol (0.18g) and ethyl 2-fluoro-α-(Z-hydroxyimino)-benzeneacetate (0.206g) in dry dichloromethane (4ml) in the presence of molecular sieves (1.5g) followed by chromatography [Kieselgel 60, hexane-ethyl acetate (19:1), then (38:1) twice] gave the title compound - (0.17g, 46%). $\nu_{max}$(film) 2940, 2865, 1740, 1455, 1265, 1215, 970cm$^{-1}$; $\delta_H$($CDCl_3$, 400MHz) 0.86 (3H, t, J7Hz), 1.2-1.47 (m, incorporating triplet 1.35, J7Hz), 1.47-1.67(m), 1.67-1.84(m), 1.95-2.10 (2H, m), 4.37 (2H, q, J7Hz), 7.02-7.12 (1H, m), 7.12-7.20 (1H, m), 7.32-7.41 (1H, m). 7.72-7.81 (1H, m); m/z CI($NH_3$ gas) $MH^+$. 378.

(b) 2-Fluoro-α-(Z-1-heptylcyclopentyloxyimino)benzeneacetic acid.

The title compound was prepared in a manner similar to that described for Example 3(d). Treatment of ethyl 2-fluoro-α-(Z-1-heptylcyclopentyloxyimino)benzeneacetate (0.13g) with 1M sodium hydroxide solution (0.78ml and 0.3ml) in ethanol (5ml) gave the title compound (0.086g, 69%). $\nu_{max}$(film) 2930, 2860, 1725, 1455, 1265, 965,m 760cm$^{-1}$; $\delta_H$($CDCl_3$) 0.84 (3H, broad triplet), 1.05-2.3(20H, m), 6.8-7.45 (3H, m), 7.45-7.75 (1H, m), 7.9 (1H, br); m/z (FAB) $MH^+$, 350.

(c) Sodium 6β-[Z-2-(2-Fluorophenyl)-2-(1-heptylcyclopentyloxyimino) acetamido]penicillanate.

Triethylamine (0.128ml) was added to 6-aminopenicillanic acid (0.099g) in dry dichloromethane (1ml) and stirred for 1.3 h. Oxalyl chloride (0.030ml) was added to N,N-dimethylformamide (0.027ml) in dry dichloromethane (1ml) at -18°C under argon, and stirred for $\frac{1}{4}$ h. 2-Fluoro-α-(Z-1-heptylcyclopentyloxyimino)benzeneacetic acid (0.08g) in dry dichloromethane (2ml) was added, and the mixture stirred for about 10 min with cooling. The triethylammonium 6-aminopenicillanate/triethylamine solution was briefly chilled, then added to the above mixture. The cooling was removed after 2 min, and the reaction mixture stirred at room temperature for $\frac{1}{2}$ h. The reaction was worked up in a similar manner to that described for Example 1(f) to give the penicillin acid. Treatment of the penicillin acid in t-butanol (10ml) and water (10ml) with dilute sodium hydroxide solution followed by partial concentration and lyophilization gave the title compound (0.101g, 77%). $\nu_{max}$(KBr) 2957, 2929, 2856, 1775, 1675, 1612, 1512, 1452, 1401, 949, 758cm$^{-1}$; $\delta_H$[250MHz, $(CD_3)_2SO$] 0.75-0.9 (3H, m), 1.0-1.85 (24H, m, incorporating peaks at 1.23, 1.46 and 1.54), 1.85-2.1 (2H, m), 3.84 (1H, s, 3-H), 5.42 (1H, d, J 3.9Hz, 5-H), 5.51 (1H, dd, J 3.9Hz and 8.3Hz, 6-H), 7.17-7.35 (2H, m), 7.40-7.63 (2H, m), 9.00 (1H, d, J8.3Hz, $D_2O$ exch., CONH); m/z (FAB), $MNa^+$, 592 and $MH^+$, 570.

## Example 15

### Sodium 6β-[Z-2-(1-Methylcyclopentyloxyimino)-2-(4-nitrophenyl)acetamido]penicillanate.

(a) Ethyl 4-Nitro-α-(Z-hydroxyimino)benzeneacetate.

The title compound was prepared in a manner similar to that described for Example 7(b). Treatment of ethyl 4-nitro-α-oxo-benzeneacetate (0.55g) in ethanol (3ml) and water (0.2ml) with hydroxylamine hydrochloride (0.18g) gave a mixture of syn- and anti-isomers. This mixture was slurried in ethyl acetate-hexane (1:3) and filtered. The filtrate was found to contain mainly the syn-isomer, and chromatography [Kieselgel 60, hexane-ethyl acetate (4:1)] gave the title compound (0.19g, 32%). (Found: $M^+$ 238.0591. $C_{10}H_{10}N_2O_5$ requires $M^+$ 238.0590). $\nu_{max}$ (KBr) 3350 (br), 1710, 1602, 1590, 1342, 1236, 1039, 957, 852cm$^{-1}$; $\delta_H$($CDCl_3$) 1.39 (3H, t, J7Hz), 4.46(2H, q, J7Hz), 7.72 and 8.22 (4H, AA'BB'q, J 8Hz), 9.13 (1H, s); m/z (EI) $M^+$, 238.

(b) Ethyl 4-Nitro-α-(Z-1-methylcyclopentyloxyimino)benzeneacetate.

The title compound was prepared in a manner similar to that described for Example 7(c). Reaction of boron trifluoride etherate (0.085ml), 1-methylcyclopentanol (0.072g), and ethyl 4-nitro-α-(Z-hydroxyimino)-benzeneacetate (0.164g) in dichloromethane (3ml) in the presence of molecular sieves (1.0g) followed by chromatography [Kieselgel 60, hexane-ethyl acetate (9:1)] gave the title compound (0.159g, 72%). (Found: $M^+$ 320.1378. $C_{16}H_{20}N_2O_5$ requires M 320.1372). $\nu_{max}$(film) 2970, 1740, 1520, 1350, 1220, 965cm$^{-1}$; $\delta_H$-(250MHz, $CDCl_3$) 1.39 (3H, t, J7Hz), 1.45-1.85 (9H, m, incorporating singlet 1.50), 1.95-2.13 (2H, m), 4.44 (2H, q, J7Hz), 7.75 and 8.23 (4H, AA'BB'q, J9Hz); m/z (EI) $M^+$, 320.

(c) 4-Nitro-α-(Z-1-methylcyclopentyloxyimino)benzeneacetic acid.

1M sodium hydroxide solution (0.96ml) was added to ethyl 4-nitro-α-(Z-1-methylcyclopentyloxyimino)-benzeneacetate (0.14g) in ethanol (3ml) and stirred at room temperature overnight. The mixture was partitioned between diethyl ether (10ml) and water (10ml). The organic phase was extracted with water (2x10ml), and the pH of the combined aqueous phase adjusted to 3.5. Following addition of ethyl acetate the pH was adjusted to 2. The organic phase was washed with water (10ml) and with saturated brine (10ml), dried over magnesium sulphate and concentrated in vacuo. The title compound was obtained after recrystallization from hexane/ethyl acetate (0.064g, 50%). (Found: C, 57.32; H, 5.22; N, 9.34%; $C_{14}H_{16}N_2O_5$ requires C, 57.53; H, 5.52; N, 9.58%); $\nu_{max}$(KBr) 1723, 1599, 1577, 1517, 1346, 1251, 966, 853cm$^{-1}$; $\delta_H$-(250MHz, $CDCl_3$) 1.55 (3H, s), 1.6-1.88 (6H, m), 2.0-2.23 (2H, m) 7.85 and 8.26 (4H, AA'BB'q, J9Hz); m/z - (EI) $M^+$, 292.

(d) Sodium 6β-[Z-2-(1-Methylcyclopentyloxyimino)-2-(4-nitrophenyl)acetamido]penicillanate.

Triethylamine (0.083ml) was added to 6-aminopenicillanic acid (0.064g) in dry dichloromethane (0.5ml) and stirred for about 1 h. Oxalyl chloride (0.019ml) was added to N,N-dimethylformamide (0.017ml) in dry dichloromethane (0.5ml) at -17°C under argon and stirred for 10 min with cooling. 4-Nitro-α-(Z-1-methylcyclopentyloxyimino)benzeneacetic acid (0.043g) in dry dichloromethane (1ml) was added, and the mixture stirred for 10 min with cooling. The chilled triethylammonium 6-aminopenicillanate/triethylamine solution was added, and after 4 min the cooling was removed and the reaction mixture stirred at room temperature for about ½ h. The reaction was worked up in a similar manner to that described for Example 1-(f) to give the penicillin acid. Treatment of the penicillin acid in t-butanol (5ml) and water (5ml) with dilute sodium hydroxide solution followed by partial concentration and lyophilization gave the title compound - (0.071, 94%), $\nu_{max}$(KBr) 1764, 1671, 1598, 1517, 1399, 1345, 962, 856cm$^{-1}$; $\delta_H$[250MHz, $(CD_3)_2SO$] 1.3-1.85 (15H, m, incorporating peaks at 1.44, 1.46 and 1.53), 1.85-2.15 (2H, m), 3.87 (1H, s, 3-H), 5.45 (1H, d, J4Hz, 5-H), 5.52 (1H, dd, J4Hz and 7.5Hz, 6-H), 7.81 and 8.30 (4H, AA''BB''q, J 9Hz), 9.49 (1H, d, J 7.5Hz, $D_2O$ exch.,CONH); m/z (FAB), $MNa^+$, 535 and $MH^+$ 513.

Example 16

Sodium 6β-[Z-2-(2-Methoxyphenyl)-2-(1-methylcyclopentyloxyimino)acetamido]penicillanate.

(a) Ethyl 2-Methoxy-α-oxo-benzeneacetate.

The title compound was prepared in a manner similar to that described for Example 8(a). Reaction of 2-methoxyphenyl magnesium bromide prepared from 2-bromoanisole (3.74g) with ethyl α-oxo-1H-imidazole-1-acetate (10.0g) followed by chromatography [Kieselgel 60, hexane-ethyl acetate (9:1)] gave the title compound (2.93g, 70%) as an oil, (Found: $M^+$ 208.0728. $C_{11}H_{12}O_4$ requires M 208.0736). $\nu_{max}$(film) 1740, 1675, 1605, 1490, 1275, 1195, 1025, 755cm$^{-1}$; $\delta_H$($CDCl_3$) 1.38 (3H, t, J7Hz), 3.91 (3H, s), 4.46 (2H, q, J 7Hz), 6.9-7.4 (2H, m), 7.5-8.2 (2H, m); m/z (EI) $M^+$, 208.

(b) Ethyl 2-Methoxy-α-(Z-hydroxyimino)benzeneacetate.

The title compound was prepared in a manner similar to that described for Example 7(b). Treatment of ethyl 2-methoxy-α-oxo-benzeneacetate (1.50g) in ethanol (9ml) and water (1ml) with hydroxylamine hydrochloride (0.5g) followed by chromatography [Kieselgel 60, hexane-ethyl acetate (1:9)-(0:1)] gave the E isomer (0.37g) and the title compound (1.07g, 66%) m.p. 77-79°C. (Found: $M^+$ 223.0841. $C_{11}H_{13}NO_4$ requires M 223.0845). $\nu_{max}$(KBr) 3307(br), 1714, 1490, 1464, 1274, 1220, 1036, 952, 758cm$^{-1}$; $\delta_H$($CDCl_3$) 1.34 (3H, t, J7Hz), 3.81 (3H, s), 4.38 (2H, q, J7Hz), 6.84-7.06,(2H, m), 7.33-7.47 (1H, m), 7.47-7.58 (1H, m), 9.91 (1H, s); m/z (EI) $M^+$, 223.

(c) Ethyl 2-Methoxy-α-(Z-1-methylcyclopentyloxyimino)benzeneacetate.

The title compound was prepared in a manner similar to that described for Example 7(c). Reaction of boron trifluoride etherate (0.437ml), 1-methylcyclopentanol (0.352g) and ethyl 2-methoxy-α-(Z-hydroxyimino)benzeneacetate (0.79g) in dichloromethane (12ml) in the presence of 3A molecular sieves (6g) followed by chromatography [Kieselgel 60, hexane-ethyl acetate (9:1)] gave a product (0.078g) consisting of a mixture of isomers, in which the required Z-isomer was predominant. The mixure (0.075g) in ethanol (1ml) was treated with 1M sodium hydroxide solution (0.074ml) and the reaction mixture stirred at room temperature for 1 h. The reaction mixture was partitioned between diethyl ether (20ml) and water (10ml), and the aqueous phase extracted with diethyl ether (10ml). The combined orgainic extracts were washed with water (10ml) and with saturated sodium chloride solution, dried over magnesium sulphate and concentrated in vacuo to give a product (0.068g) which still contained some E-isomer. Treatment of this material with 1M sodium hydroxide solution (0.045ml) in ethanol (0.5ml) in similar manner to that described above gave the title compound (0.048g, 4%). (Found: $M^+$ 305.1624. $C_{17}H_{23}NO_4$ requires M 305.1627.) $\nu_{max}$(film) 1735, 1605, 1490, 1465, 1275, 1215, 1035, 960, 755cm$^{-1}$; $\delta_H$($CDCl_3$) 1.2-1.86 (m, incorporating triplet 1.36, J7Hz, and singlet 1.47), 1.95-2.15 (2H, m), 3.79 (3H, s), 4.35 (2H, q, J7Hz), 6.83-7.05 (2H, m), 7.30-7.43 (1H, m), 7.67-7.80 (1H, m); m/z (EI) $M^+$, 305.

(d) 2-Methoxy-α-(Z-1-methylcyclopentyloxyimino)benzeneacetic acid.

The title compound was prepared in a manner similar to that described for Example 3(d). Treatment of ethyl 2-methoxy-α-(Z-1-methylcyclopentyloxyimino)benzeneacetate (0.038g) with 1M sodium hydroxide solution (0.31ml and 0.15ml) in ethanol (1ml) gave the title compound (0.025g, 71%). $\nu_{max}$ (KBr) 1705, 1601, 1488, 1281, 1027, 960cm$^{-1}$; $\delta_H$ ($CDCl_3$, $D_2O$ shake) 1.52 (3H, s), 1.56-1.87 (6H, m) 2.00-2.20 (2H, m), 3.82 (3H, s), 6.87-7.06 (2H, m), 7.33-7.50 (2H, m); m/z (EI) $M^+$, 277.

(e) Sodium 6β-[Z-2-(2-Methoxyphenyl)-2-(1-methylcyclopentyloxyimino)acetamido]penicillanate

Triethylamine (0.048ml) was added to 6-aminopenicillanic acid (0.038g) in dry dichloromethane (0.5ml) and stirred for 1h. Oxalyl chloride (0.011ml) was added to N,N-dimethylformamide (0.010ml) in dry dichloromethane (0.25ml) at -17°C under argon, and stirred for 10min. 2-Methoxy-α-(Z-1-methylcyclopentyloxyimino)benzeneacetic acid (0.024g) in dry dichloromethane (1ml) was added and the mixture stirred for 12min. with cooling. The triethylammonium 6-aminopenicillanate/triethylamine solution was added to the above mixture, and after 5min. the cooling was removed and the reaction stirred at room temperature for $\frac{1}{2}$ h. The reaction was worked up in a similar manner to that described for Example 1(f) to give the penicillin acid. Treatment of the penicillin acid in t-butanol (5ml) and water (5ml) with dilute sodium hydroxide solution followed by partial concentration and lyophilisation gave the title compound (0.038g, 88%). $\nu_{max}$ (KBr) 1774, 1675, 1602, 1515, 1401, 1026, 945, 753cm$^{-1}$; $\delta_H$ [250MHz, $(CD_3)_2SO$] 1.2-1.85 (15H, m, incorporating peaks

at 1.40, 1.46 and 1.54), 1.85-2.15 (2H, m), 3.72 (3H, s), 3.86 (1H, s), 5.45 (1H, d, J 4Hz), 5.56 (1H, dd, J 4Hz and 9Hz), 6.9-7.14 (2H, m), 7.33-7.50 (2H, m), 8.26 (1H, d, J 9Hz $D_2O$ exch., CONH); m/z (FAB) $MH^+$, 498).

Example 17

a) 3-Trifluoromethylphenylglyoxylic acid.

3-Trifluoromethyl acetophenone (18.2g, 0.1mol) in pyridine (100ml) was stirred and treated with selenium dioxide (15g). The mixture was stirred and heated at 70° for 6h. then cooled and filtered. The filtrate was poured onto crushed ice (400g) and acidified with conc. hydrochloric acid. The mixture was extracted exhaustively with ethyl acetate. The combined extracts were dried and evaporated to give an oil which crystallised 19.2g (88%). Recrystallisation from benzene gave a sample mp. 89-90°C. Found: C, 49.52; H, 2.36. $C_9H_5F_3O_3$ required: C, 49.55, H, 2.31%.

b) α-Cyclopentyloxyimino-3-trifluoromethylphenylacetic acid.

A suspension of 3-trifluoromethylphenylglyoxylic acid (4.36g, 0.02mol) and O-cyclopentylhydroxylamine hydrochloride (3.04g 0.022mol) in water (40ml) was stirred and treated with ethanol (30ml). The mixture was adjusted to pH 4-5 with 10% sodium hydroxide and stirred at this pH for 3h at RT. The solution was adjusted to pH8 with 10% sodium hydroxide and evaporated to remove ethanol. The residue was diluted with water, washed with ether and filtered to clarify. The clear aqueous layer was acidified to give an oil. The crude product was extracted with ether. The combined extracts were dried and evaporated to give the required product as an oil 5.69g (98%).

c) Sodium (Z)-α-Cyclopentyloximino-3-trifluoromethylbenzyl penicillanate.

α-Cyclopentyloxyimino-3-trifluoromethylphenyl acetic acid (1.156g. 0.004mol) in methanol (20ml) was treated with a solution of sodium (0.094g) in methanol (20ml) and evaporated. The residue was dried in vacuo. The solid sodium salt (0.653g, 0.002mol) in dry benzene (10ml) and dimethylformamide (3 drops) was treated with oxalyl chloride (0.17ml) and stirred at RT for 1h. The mixture was evaporated and the residue in methylene dichloride (30ml) was added to a solution of 6-aminopenicillanic acid triethylammonium salt (0.64g, 0.002mol) in methylene chloride (30ml) with triethylamine (0.6ml) cooled to 0°. The mixture was stirred 1hr at RT and evaporated. The residue mixed with water (20ml), was washed with ether, layered with fresh ether and adjusted to pH2. The organic layer was separated, dried and treated with 2N sodium 2-ethyl hexanoate in methyl isobutylketone (1ml). The total solvent was evaporated and the residue triturated with n-hexane. The solid penicillin sodium salt was filtered, washed with n-hexane and dried in vacuo to give the crude penicillin sodium salt 0.67g. (66%). The solid was dissolved in water and purified on HP20SS, using water/acetone mixtures. The relevant fractions were combined, evaporated to remove acetone and lyophilised to give the title compound as a white amorphous solid (0.24g). $\delta[(CD_3)_2SO]$ 1.47 and 1.53 (6H, 2s), 1.5-2.0 (8H, m), 3.90 (1H, s), 4.79 (1H, m), 5.49 (2H, m), 7.60-7.95 (4H, m, aromatic protons), 9.42 (1H, m).

Example 18

a) 2-Fluorophenylglyoxylic acid.

2-Fluoroacetophenone (13.8g. 0.1mol) was oxidised to the title compound as described in Example 17a. The product 13.6g (81%) was purified by trituration with toluene to give a crystalline solid mp. 70-73°C. $\nu_{max}$ (Nujol) 1705, 1680, 1605$cm^{-1}$.

b) α-Cycloheptyloxyimino-2-fluorophenylacetic acid.

2-Fluorophenylglyoxylic acid (2.52g. 0.015mol) was reacted with O-cycloheptylhydroxylamine hydrochloride (2.72g) as described in Example 17b. The title compound was obtained as an oil 4.14g (98%) which slowly crystallised. Recrystallisation from cyclohexane gave a colourless crystalline solid mp 110-111°C. Found: C,65.22; H, 6.41; N, 4.86. $C_{15}H_{18}FNO_3$ required: C, 64.50; H, 6.50; N, 5.02%.

c) Sodium (Z)-α-Cycloheptyloximino-2-fluorobenzylpenicillanate.

The crude semi-crystalline α-cycloheptyloxyimino-2-fluorophenylacetic acid converted to the sodium salt (0.6g, 0.002mol), was coupled with triethylammonium 6-aminopenicillanate (0.64g, 0.002mol) as described in Example 17c to give the title compound as a white freeze-dried solid 0.21g. δ[$(CD_3)_2SO$] 1.48 and 1.55 (6H, 2s), 1.30-2.00 (12H, m), 3.89 (1H, s), 4.32 (1H, m), 5.50 (2H, m), 7.00-7.70 (4H, m), 9.05 (1H, m, exch).

Example 19

a) α-Methoxymino-3-trifluoromethylphenylacetic acid.

A solution of sodium (1g. 0.044mol) in methanol (20ml) was added dropwise with stirring to a solution of methoxyamine hydrochloride (3g. 0.036mol) until alkaline to phenolphthalein. A crystal of methoxyamine hydro- chloride was added to discharge the colour. The mixture was filtered and the clear filtrate added to a solution of 3-trifluoromethylphenylglyoxylic acid (6.5g, 0.03mol) in methanol (30ml) and refluxed for 2h. The solution was evaporated and the residue triturated with dry ether. The mixture was filtered and the filtrate evaporated to give the crude product as an oil 7.22g (97%). On standing, crystallisation began to occur. A sample recrystallised from ethanol/water had mp. 114-115°C. δ[$(CD_3)_2SO$] 3.94 (3H, s), 7.70 (4H, m). Found: C, 48.49; H, 3.28; N, 5.60. $C_{10}H_8F_3NO_3$ requires: C, 48.59; H, 3.26; N, 5.67%.

b) Sodium (Z)-α-Methoxyimino-3-trifluoromethyl-benzylpenicillanate.

Crude oily α-methoxyimino-3-trifluoromethylphenyl acetic acid converted to the sodium salt (0.54g, 0.002mol) was coupled with triethylammonium 6-aminopenicillanate (0.64g, 0.002mol) as described in Example 17c to give the title compound as a white freeze-dried solid 0.16g. δ[$(CD_3)_2SO$] 1.47 and 1.50 (6H, 2s), 3.89 (1H, s), 3.92 (3H, s), 5.46 (2H, m), 7.50-7.95 (4H, m), 9.58 (1H, m, exch.).

Example 20

Sodium (Z)-α-Cyclohexyloxyimino-3-trifluomethylbenzyl penicillanate.

α-Cyclohexyloxy-3-trifluoromethylphenylacetic acid was prepared from 3-trifluoromethylphenylglyoxylic acid and O-cyclohexylhydroxylamine hydrochloride as described in Example 17b, and then coupled with triethylammonium 6-aminopenicillanate as described in Example 17c to give the title compound.
δ [$CD_3)_2SO$] 1.49 abd 1.52 (6H, 2s) 1.1-2.1 (10H, m), 3.89 (1H, s), 4.1-4.4 (1H. m), 5.50 (2H, m), 7.46-7.95 (4H, m), 9.45 (1H, m, exch.)

Example 21

Sodium (Z)-α-Cyclohexyloxyimino-2-fluorobenzyl penicillanate.

2-Fluorophenylglyoxylic acid prepared as described in Example 18a was converted to the title compound using the procedure of Example 20.
δ[$CD_3)_2SO$] 1.47 and 1.53 (6H, 2s), 1.1-2.05 (10H, m), 3.88 (1H, s), 4.0-4.3 (1H, m) 5.50 (2H, m), 7.05-7.75

(4H, m), 9.05 (1H, d, exch.).

Example 22

a) α-t-Butyloximino-2-fluorophenylacetic acid.

2-Fluorophenylglyoxylic acid (1.68g 0.01mol) was reacted with O-t-butylhydroxylaminehydrochloride (1.38g 0.011mol), as described in Example 17b, to give the title compound (1.74g 72.8%). Recrystallisation from 60-80° petroleum ether gave a colourless crystalline solid mp 95-7° C.
Found: C, 60.07: H, 5.87; N, 5.85. $C_{12}H_{14}FNO_3$ required: C, 60.24; G, 5.90; N, 5.86%.

b) Sodium (Z) α-t-Butyloximino-2-fluorobenzylpenicillanate

The crude α-t-butyloximino-2-fluorophenylacetic acid, converted to the sodium salt (0.52g 0.002mol), was coupled with triethylammonium 6-aminopenicillanate (0.64g 0.002mol) as described in Example 17c to give the title compound as a white freeze dried solid 0.11g. δ[$(CD_3)_2SO$] 1.30 (9H, s), 1.48 and 1.56 (6H, 2s), 3.91 (1H, s), 5.50 (2H, m), 7.0-7.70 (4H, m), 8.96 (1H, d, exch).

## Claims

1. A compound of formula (I) or a pharmaceutically acceptable salt or in-vivo hydrolysable ester thereof:

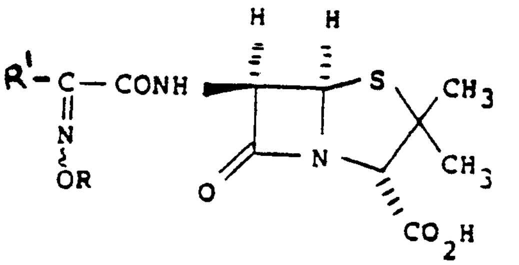

(I)

wherein $R^1$ is optionally substituted phenyl and R is a cycloalkyl group having an alkyl substituent in the 1-position; or $R^1$ is phenyl substituted by at least one fluorine atom and/or at least one trifluoromethyl group and R is hydrogen or an organic radical.

2. A compound according to claim 1, wherein $R^1$ is phenyl substituted by at least one fluorine atom and/or at least one trifluoromethyl group and R is a cycloalkyl group having an alkyl substituent in the 1-position.

3. A compound according to claim 2, wherein R is a $C_{5-7}$ cycloalkyl group having a methyl or ethyl substituent in the 1-position.

4. A compound according to claim 1, selected from the group consisting of:
6β-[Z-2-(2-fluorophenyl)-2-(1-methylcyclopentyloxyimino)acetamido]penicillanic acid,
6β-[Z-2-(2-fluorophenyl)-2-(1-methylcyclohexyloxyimino)acetamido]penicillanic acid,
6β-[Z-2-(2-fluorophenyl)-2-(1-methylcycloheptyloxyimino) acetamido]penicillanic acid,
6β-[Z-2-(1-methylcyclopentyloxyimino)-2-phenylacetamido]penicillanic acid,
6β-[Z-2-(1-methylcyclopentyloxyimino)-2-(4-trifluoromethylphenyl)acetamido]penicillanic acid,
6β-[Z-2-(2,4-difluorophenyl)-2-(1-methylcyclohexyloxyimino)acetamido]penicillanic acid,
6β-[Z-2-(1-ethylcyclopentyloxyimino)-2-(2-fluorophenyl)acetamido]penicillanic acid,
6β-[Z-2-(4-chlorophenyl)-2-(1-methylcyclopentyloxyimino)acetamido]penicillanic acid,
6β-[Z-2-(4-fluorophenyl)-2-(1-methylcyclopentyloxyimino)acetamido]penicillanic acid,
6β-[Z-2-(2,6-difluorophenyl)-2-(1-methylcyclopentyloxyimino)acetamido]penicillanic acid,
6β-[Z-2-(3-fluorophenyl)-2-(1-methylcyclopentyloxyimino)acetamido]penicillanic acid,
6β-[Z-2-(2-chloro-6-fluorophenyl)-2-(1-methyl cyclopentyloxyimino)acetamido]penicillanic acid,
6β-[Z-2-(2-fluorophenyl)-2-(1-methylcyclooctyl oxyimino)acetamido]penicillanic acid,

6β-[Z-2-(2-fluorophenyl)-2-(1-heptylcyclopentyloxyimino)acetamido]penicillanic acid,
6β-[Z-2-(1-methylcyclopentyloxyimino)-2-(4-nitrophenyl)acetamido]penicillanic acid,
6β-[Z-2-(2-methoxyphenyl)-2-(1-methylcyclopentyloxyimino)acetamido]penicillanic acid,
6β-[Z-2-(2-fluorophenyl)-2-(cycloheptyloxyimino)acetamido]penicillanic acid,
6β-[Z-2-(3-trifluoromethylphenyl)-2-(methoxyimino)acetamido]penicillanic acid,
6β-[Z-2-(3-trifluoromethylphenyl)-2-(cyclohexyloxyimino)acetamido]penicillanic acid,
6β-[Z-2-(2-fluorophenyl)-2-(cyclohexyloxyimino)acetamido]penicillanic acid, and
6β-[Z-2-(2-fluorophenyl)-2-(t-butyloxyimino)acetamido]penicillanic acid. and pharmaceutically acceptable salts and in-vivo hydrolysable esters thereof.

5. A process for the preparation of a compound according to claim 1, which process comprises treating a compound of formula (III) or salt thereof:

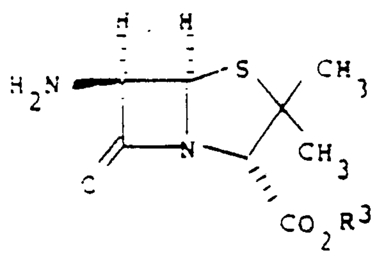

(III)

wherein the amino group is optionally substituted with a group which permits acylation to take place, and $R^3$ is hydrogen or a readily removable carboxyl blocking group; with an acylating agent derived from the acid of formula (IV):

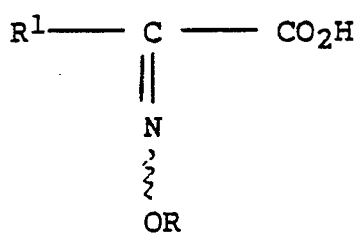

(IV)

wherein R and $R^1$ are as defined with respect to formula (I).

6. A compound of formula (IV) as defined in claim 5, or an ester, salt, or acylating derivative thereof.

7. A pharmaceutical composition which comprises a compound according to any one of claims 1 to 4, and a pharmaceutically acceptable carrier.

8. A compound according to any one of claims 1 to 4, for use in therapy.

9. A compound according to any one of claims 1 to 4, for use in the treatment of bacterial infections in humans and animals.

10. Use of a compound according to any one of claims 1 to 4 in the manufacture of a medicament for use in the treatment of bacterial infections in humans and animals.

Claims for the following Contracting State:ES

1. A process for the preparation of a compound of formula (I) or a pharmaceutically acceptable salt or in-vivo hydrolysable ester thereof:

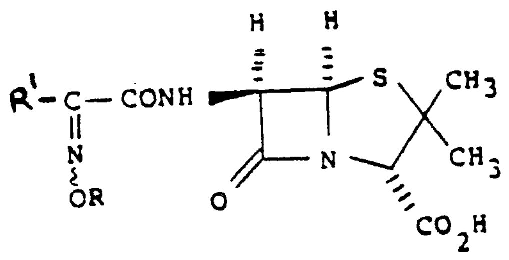

(I)

wherein $R^1$ is optionally substituted phenyl and R is a cycloalkyl group having an alkyl substituent in the 1-position; or $R^1$ is phenyl substituted by at least one fluorine atom and/or at least one trifluoromethyl group and R is hydrogen or an organic radical, which process comprises treating a compound of formula (III) or salt thereof:

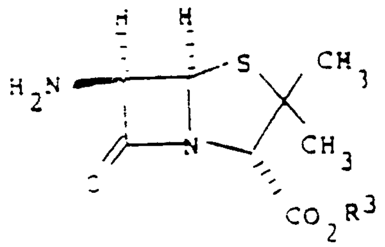

(III)

wherein the amino group is optionally substituted with a group which permits acylation to take place, and $R^3$ is hydrogen or a readily removable carboxyl blocking group; with an acylating agent derived from the acid of formula (IV):

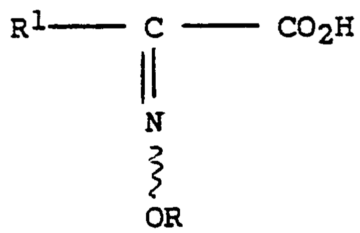

(IV)

wherein R and $R^1$ are as defined with respect to formula (I).

2. A process according to claim 1, wherein $R^1$ is phenyl substituted by at least one fluorine atom and/or at least one trifluoromethyl group and R is a cycloalkyl group having an alkyl substituent in the 1-position.

3. A process according to claim 2, wherein R is a $C_{5-7}$ cycloalkyl group having a methyl or ethyl substituent in the 1-position.

4. A process according to claim 1, for the preparation of a compound selected from the group consisting of:

6β-[Z-2-(2-fluorophenyl)-2-(1-methylcyclopentyloxyimino)acetamido]penicillanic acid,
6β-[Z-2-(2-fluorophenyl)-2-(1-methylcyclohexyloxyimino) acetamido]penicillanic acid,
6β-[Z-2-(2-fluorophenyl)-2-(1-methylcycloheptyloxyimino) acetamido]penicillanic acid,
6β-[Z-2-(1-methylcyclopentyloxyimino)-2-phenylacetamido]penicillanic acid,
6β-[Z-2-(1-methylcyclopentyloxyimino)-2-(4-trifluoromethylphenyl)acetamido]penicillanic acid,
6β-[Z-2-(2,4-difluorophenyl)-2-(1-methylcyclohexyloxyimino)acetamido]penicillanic acid,
6β-[Z-2-(1-ethylcyclopentyloxyimino)-2-(2-fluorophenyl)acetamido]penicillanic acid,
6β-[Z-2-(4-chlorophenyl)-2-(1-methylcyclopentyloxyimino)acetamido]penicillanic acid,
6β-[Z-2-(4-fluorophenyl)-2-(1-methylcyclopentyloxyimino)acetamido]penicillanic acid,
6β-[Z-2-(2,6-difluorophenyl)-2-(1-methylcyclopentyloxyimino)acetamido]penicillanic acid,
6β-[Z-2-(3-fluorophenyl)-2-(1-methylcyclopentyloxyimino)acetamido]penicillanic acid,

6β-[Z-2-(2-chloro-6-fluorophenyl)-2-(1-methyl cyclopentyloxyimino)acetamido]penicillanic acid,
6β-[Z-2-(2-fluorophenyl)-2-(1-methylcyclooctyl oxyimino)acetamido]penicillanic acid,
6β-[Z-2-(2-fluorophenyl)-2-(1-heptylcyclopentyloxyimino)acetamido]penicillanic acid,
6β-[Z-2-(1-methylcyclopentyloxyimino)-2-(4-nitrophenyl)acetamido]penicillanic acid,
6β-[Z-2-(2-methoxyphenyl)-2-(1-methylcyclopentyloxyimino)acetamido]penicillanic acid,
6β-[Z-2-(2-fluorophenyl)-2-(cycloheptyloxyimino)acetamido]penicillanic acid,
6β-[Z-2-(3-trifluoromethylphenyl)-2-(methoxyimino)acetamido]penicillanic acid,
6β-[Z-2-(3-trifluoromethylphenyl)-2-(cyclohexyloxyimino)acetamido]penicillanic acid,
6β-[Z-2-(2-fluorophenyl)-2-(cyclohexyloxyimino)acetamido]penicillanic acid, and
6β-[Z-2-(2-fluorophenyl)-2-(t-butyloxyimino)acetamido]penicillanic acid,
and pharmaceutically acceptable salts and in-vivo hydrolysable esters thereof.

5. A compound of formula (IV) as defined in claim 1, or an ester, salt, or acylating derivative thereof.

6. A pharmaceutical composition which comprises a compound of formula (I) as defined in claim 1, or a pharmaceutically acceptable salt or in-vivo hydrolysable ester thereof, and a pharmaceutically acceptable carrier.

7. Use of a compound of formula (I) as defined in claim 1, or a pharmaceutically acceptable salt or in-vivo hydrolysable ester thereof, in the manufacture of amedicament for use in the treatment of bacterial infections in humans and animals.

European Patent Office

# EUROPEAN SEARCH REPORT

Application Number

EP 90 30 2187

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-3 971 778 (M.C. COOK et al.)<br>* Columns 39-40; preparation no, 48; columns 67-68, example no. 72; columns 1-2 *<br>--- | 1,5-10 | C 07 D 499/68<br>C 07 C 251/48<br>A 61 K 31/43 |
| Y | EP-A-0 254 426 (I.C.I.)<br>* Claims *<br>--- | 6 | |
| D,Y | GB-A-1 399 087 (GLAXO)<br>* Claims *<br>----- | 1,5-10 | |
| | | | TECHNICAL FIELDS SEARCHED (Int. Cl.5) |
| | | | C 07 D 499/00<br>C 07 C 251/00<br>A 61 K 31/00 |

The present search report has been drawn up for all claims

| Place of search | Date of completion of the search | Examiner |
|---|---|---|
| THE HAGUE | 01-06-1990 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document